# EUROPEAN PATENT SPECIFICATION

(11) **EP 4 410 950 B1**
(45) Date of publication and mention of the grant of the patent: **28.05.2025**
(21) Application number: 23154610.2
(22) Date of filing: 02.02.2023
(51) Int. Cl.: C12M 1/36

(54) **GRAVITATIONAL LIQUID-SPLITTING DEVICE, CELL PASSAGING DEVICE WITH THE SAME, AND CELL PASSAGING METHOD**
SCHWERKRAFTFLÜSSIGKEITSTEILUNGSVORRICHTUNG, ZELLENDURCHGANGSVORRICHTUNG DAMIT UND ZELLENDURCHGANGSVERFAHREN
DISPOSITIF GRAVITATIONNEL DE SÉPARATION DE LIQUIDES, DISPOSITIF DE PASSAGE DE CELLULES DOTÉ DE CELUI-CI ET PROCÉDÉ DE PASSAGE DE CELLULES

(43) Date of publication of application: 07.08.2024
(73) Proprietor: Drsignal Biotechnology Co., Ltd, Neihu Dist Taipei City (TW)
(72) Inventor: MI, Hsin-Wu, Taipei City (TW); LIN, Chih-Huang, Taipei City (TW); HUANG, Hsin-Fei, Taipei City (TW); HSU, Chia-I, Taipei City (TW)
(74) Representative: Melchior, Robin

(56) References cited:
- WO-A1-2020/247832
- US-A1- 2006 257 999
- US-A1- 2017 226 465
- US-A1- 2020 025 782
- EGGERT ARMIN ET AL: "Liquid-liquid centrifugal separation - New equipment for optical (photographic) evaluation at laboratory scale", CHEMICAL ENGINEERING RESEARCH AND DESIGN, vol. 127, 12 September 2017 (2017-09-12), pages 170 - 179, XP085242391, ISSN: 0263-8762, DOI: 10.1016/J.CHERD.2017.09.005

## Description

### BACKGROUND

### 1. Field of the Invention

The present invention relates to a cell passaging device, especially to a device that is configured to split liquid or liquid with cells equally between different containers using gravity.

### 2. Description of the Prior Arts

Cell passaging is done when the cultured cells, such as human stem cells, are 90%-100% confluent. First, an enzyme and related solution(s) are added to the culture vessel to make the cultured cells detach from a surface of the culture vessel, and then the solution with detached cells (hereinafter referred to as "cell solution") are equally dispensed into double centrifuge tubes, and finally the detached cells are separated from the solutions by using a centrifuge. The cells separated by the centrifuge are transferred to different kinds of containers for subsequent cell passaging process or other processes such as cryo-preservation or bottling for shipping.

When manually dispensing the cell solution to the centrifuge tubes, the cell solution must be split equally for the double centrifuge tubes either volume-wise or weight-wise to balance the centrifuge. The existing manual procedure for splitting the cell solution for the double centrifuge tubes is too complicated and time consuming, and therefor is unsuitable and troublesome for directly converting to an automated process.

Peristaltic pumps are generally used to dispense the liquid or cell solution into the containers, such as centrifuge tubes. Though the peristaltic pumps are capable of precisely controlling a flow of the liquid or the cell solution, a part of the cell solution is stranded in the pump due to significant length of the tubing in the pump, and therefore some of the cells are lost each time when the cell solution is dispensed by the pump.

However, a total amount of the cell solution to be transferred for the cell subculture is limited, and each milliliter of the cell solution comprises a significant amount of cells; therefore, using the peristaltic pump to dispense the cell solution results in loss of a great deal of precious cells.

Moreover, in order to dispense the cell solution, the peristaltic pump inevitably has to pressurize the cell solution, and the pressure difference in the peristaltic pump causes damage to the cells. As a result, the use of the peristaltic pump reduces efficiency of the cell passaging and increases cost of cell passaging.

Additionally, several steps need to be performed before transferring the cell solution to different containers in the cell passaging process. For adherent cells, an enzyme and related solution(s) has to be added to make the cells detach from the surface of the culture vessel. However, currently all steps are performed manually, and are therefore time-consuming, difficult to control quality; risk of contamination due to improper handling is also needed to take into consideration.

In view of the problems of the conventional cell passaging, when using automated equipment to transfer a solution with detached cells, the solutions with detached cells from multiple cell culture containers are first collected in a specialized container, and then a gravitational liquid-splitting device is used to split the collected solution with detached cells equally between two centrifuge containers, and then the two centrifuge containers are disposed symmetrically in a centrifuge for centrifugation, thereby ensuring dynamic balance during centrifugation.

US 2017/226465 discloses an apparatus and a method to prepare cell suspensions for clinical use, describing that the cell culture vessel is coupled to the culture medium supply part at a position lower in the direction of gravitational force than the culture medium supply part.

### SUMMARY OF THE INVENTION

The main objective of the present invention is to provide a gravitational liquid-splitting device, a cell passaging device with the same, and a cell passaging method to mitigate loss of cells when the solution with detached cells is being transferred to different containers.

The gravitational liquid-splitting device has a funnel, a dispensing valve, a liquid-splitting part, and a stirring assembly. The funnel has an inner space and a bottom opening connected to the bottom of the funnel. The dispensing valve is mounted to the funnel and is configured to close or open the bottom opening of the funnel. The liquid-splitting part is connected to a bottom of the funnel and has an input channel and two output channels. The input channel is formed in the liquid-splitting part and extends upward and downward. An upper end of the input channel forms an input opening on a top of the liquid-splitting part and is connected to the bottom opening of the funnel. The two output channels are formed in the liquid-splitting part. Each of the output channels has a first end and a second end. The first end is connected to a lower end of the input channel. The second end extends inclinedly downward and is connected to an exterior of the liquid-splitting part. The stirring assembly has a funnel and a stirrer. The funnel cover detachably covers an upper opening of the funnel. The stirrer is connected to the funnel cover and is configured to stir liquid in the funnel.

The cell passaging device is configured to dispense contents collected from multiple cell culture containers into two centrifuge containers by gravity. The cell passaging device has a base, a container-handling device, and a liquid equal-splitting device. The container-handling device is mounted on the base and has a container-positioning table, multiple container holders, and multiple container-driving assemblies. The container-positioning table is rotatably or movably mounted on the base. The container holders are pivotally mounted on the container-positioning table. Each of the container holders is configured to accommodate one of the cell culture containers or one of the centrifuge containers, and is pivotable to an emptying angle to empty contents in the cell culture container or the centrifuge container. Each of the container-driving assemblies controls an angle of a respective one of the container holders and is configured to rotate the respective one of the container holders to the emptying angle or to sway the respective one of the container holders. The container-positioning table is configured to move each of the container holders to a first injection position. The liquid equal-splitting device is disposed on a side of the container-handling device. The liquid equal-splitting device has a funnel-positioning table and at least one said gravitational liquid-splitting device as mentioned above. The funnel-positioning table is rotatably or movably mounted on the base. The at least one gravitational liquid-splitting device is mounted on the funnel-positioning table. The funnel-positioning table is configured to move the at least one gravitational liquid-splitting device from a position under the first injection position to a position above the two centrifuge containers such that liquid in the funnel of the at least one gravitational liquid-splitting device flows into the two centrifuge containers via the two output channels respectively.

The cell passaging method comprises: using the gravitational liquid-splitting device to split contents collected from the multiple cell culture containers equally between the two centrifuge containers, and then dispose the two centrifuge containers oppositely in a centrifuge and use the centrifuge to perform centrifugation to separate the cells from the solution.

When using the cell passaging device, first add a solution, which can detach cells from the surface of the cell culture container, into the cell culture container. Then, sway the cell culture container using the container-handling device to uniformly distribute the solution in the cell culture container and make the cells detach from the surface of the cell culture container. Then, rotate the culture container to an emptying angle using the container-handling device to make the cell solution in the cell culture container flow into the gravitational liquid-splitting device located under the cell culture container. Subsequently, the cell solution automatically flows downward and is split into two equal portions by the liquid-splitting part due to gravity, and the two equal portions enter the two centrifuge containers respectively.

The advantages of the present invention are as follows:
First, liquid in the funnel automatically flows downward into the centrifuge containers due to gravity because the liquid-splitting part is disposed under the funnel, the input channel extends upward and downward, and the output channels extend inclinedly downward. Therefore, the present invention prevents loss of cells during transferring operation, avoids damage to the cells under pressurization by using pump, and effectively automates the process of collecting solution with detached cells that are already separated from the cell culture containers together and then split the collected solution with detached cells equally between the two centrifuge containers. As a result, the present invention improves efficiency of cell passaging, increases cell survival rate of cell passaging, and reduces cost of cell passaging.

Second, because the stirrer can continuously stir the solution with detached cells in the funnel, the present invention prevents cells in the solution from clustering together or adhering to surfaces of the channels, thereby keeping flow rates in the two output channels remain equal and ensuring the solution with detached cells is split equally between the two centrifuge containers.

Third, the cell passaging device replaces traditional manual operation by automatically swaying the cell culture container to efficiently detach the adherent cells from the surface of the container, transferring the solution with detached cells into the gravitational liquid-splitting device, and splitting the solution with detached cells equally between the two centrifuge containers, thereby reducing labor-intensity in the cell passaging operation. As a result, the present invention has advantages such as high efficiency, stable quality, and reducing risk of contamination of cell passaging processes.

### BRIEF DESCRIPTION OF THE DRAWINGS

Fig. 1 is a perspective view of a cell passaging device in accordance with the present invention;
Fig. 2 is another perspective view of the cell passaging device in Fig. 1, showing an internal structure of the cell passaging device;
Fig. 3 is a top view of the internal structure of the cell passaging device in Fig. 2;
Fig. 4 is a simplified schematic top view of the internal structure of the cell passaging device in Fig. 3;
Fig. 5 is an exploded perspective view of a container-handling device and a primary container-moving device of the cell passaging device in Fig. 1;
Fig. 6 is a perspective view of the container-handling device of the cell passaging device in Fig. 1;
Fig. 7 is an enlarged exploded perspective view of the container-handling device of the cell passaging device in Fig. 6;
Figs. 8 to 10 are enlarged side views of the cell passaging device in Fig. 1, showing a first multi-axis transfer mechanism of the primary container-moving device moving a cell culture container to a container-handling device;
Fig. 11 is another enlarged side view of the cell passaging device in Fig. 1, showing a cap mechanism moving down and gripping a cap of a cell culture container;
Fig. 12 is another enlarged side view of the cell passaging device in Fig. 1, showing an injection head of a liquid solution injection device aligned with the cell culture container;
Fig. 13 is another enlarged side view of the cell passaging device in Fig. 1, showing the cell culture container being swayed by the container-handling device;
Figs. 14 to 16 are enlarged schematic top views of the cell passaging device in Fig. 1, showing an auxiliary container-moving device moving the cell culture container in a container output position, to a centrifuge, or to an optical inspection device;
Fig. 17 is an enlarged exploded perspective view of the cell passaging device in Fig. 1, showing a liquid equal-splitting device;
Fig. 18 is an exploded perspective view of the liquid equal-splitting device of the cell passaging device Fig. 17;
Fig. 19 is an exploded perspective view of a gravitational liquid-splitting device of the cell passaging device Fig. 18;
Fig. 20 is a sectional view of the gravitational liquid-splitting device of the cell passaging device Fig. 19, showing a first ferromagnetic part closing a bottom opening of a funnel;
Figs. 21 and 22 are enlarged sectional views of the container-handling device of the cell passaging device in Fig. 1, showing the cell culture container being rotated to an emptying angle to transfer contents of the cell culture container to the gravitational liquid-splitting device thereunder;
Figs. 23 and 24 are enlarged side views of the container-handling device of the cell passaging device in Fig. 1, showing a stirring motor moved downward to connect a stirrer;
Fig. 25 is an enlarged sectional view of the cell passaging device in Fig. 1, showing a dispensing valve opening the bottom opening of the funnel;
Fig. 26 is another enlarged exploded perspective view of the cell passaging device in Fig. 1;
Fig. 27 is another enlarged side view of the cell passaging device in Fig. 1; and
Fig. 28 is another enlarged sectional view of the cell passaging device in Fig. 1, showing contents in the gravitational liquid-splitting device being split equally and flowing downward to two centrifuge containers thereunder.

### DETAILED DESCRIPTION OF THE INVENTION

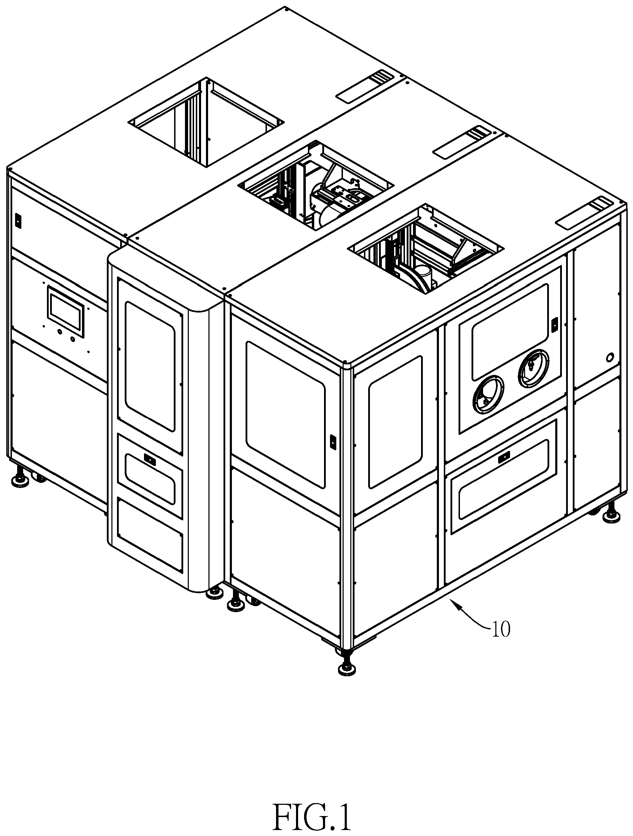

With reference to Figs. 1 to 4 and 28, the cell passaging device in accordance with the present invention is configured to collect contents (i.e. solution with detached cells) from multiple cell culture containers 91, and then split the collected contents between the two centrifuge containers 92 for centrifugation. Cells in solution separated by centrifuge can be used for cell subculture with other cell culture devices. The cell passaging is preferably also configured to transfer the separated cells from solution to cryovials (not shown in figures) for cryopreservation, or to cell solution containers (not shown in figures) for shipments.

The cell culture containers 91 and the centrifuge containers 92 are containers in the same shape preferably. In the preferred embodiment, the culture containers 91 are configured to be put in a centrifuge directly for centrifugation, and therefore the centrifuge containers 92 can be two of the cell culture containers 91 as well.

The cell passaging device has a base 10, a container-handling device 20, and a liquid equal-splitting device 40. In the preferred embodiment, the cell passaging device further has a liquid solution injection device 30, a primary container-moving device 50, an auxiliary container-moving device 60, a centrifuge 70, and an optical inspection device 80.

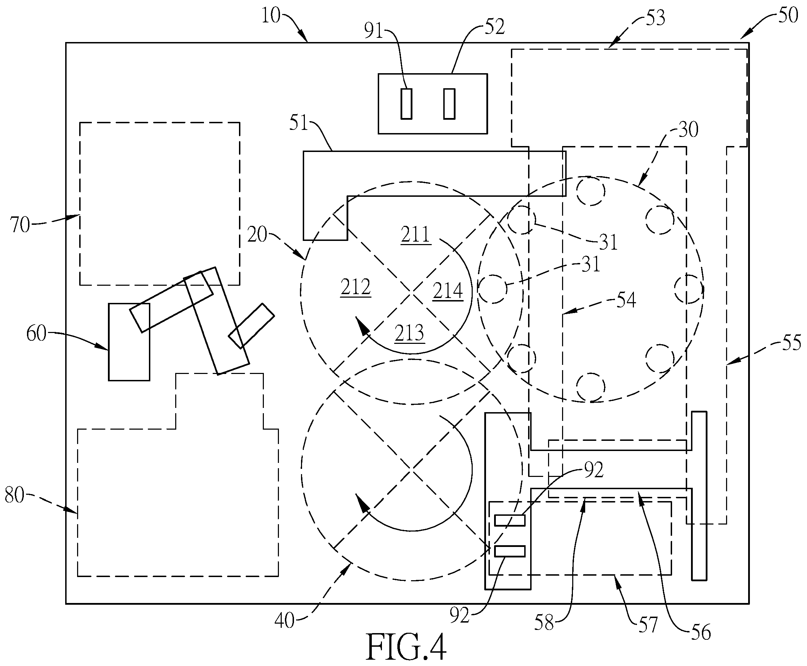

With reference to Figs. 4 to 7, the container-handling device 20 is mounted on the base 10 and has a container-positioning table 21, multiple container holders 22, and multiple container-driving assemblies 23.

The container-positioning table 21 is rotatably mounted on the base 10, and is preferably a conventional rotary indexing table. The container-positioning table 21 rotates 90 degrees each time, thereby defining four stationary positions that are fixed in space. The four stationary positions are respectively a container-receiving position 211, a container output position 212, a first injection position 213, and a second injection position 214, wherein the second injection position 214 and the container output position 212 are oppositely disposed on the container-handling device 20.

In the terminology of this technical field, the container-positioning table 21 has four indexed positions: the aforementioned container-receiving position 211, the container output position 212, the first injection position 213, and the second injection position 214 are the four indexed positions of the container-positioning table 21.

A number of the container holders 22 is preferably four; the four container holders 22 are disposed around the container-positioning table 21 at angular intervals of 90 degrees. The container-positioning table 21 is configured to move each container holder 22 cyclically among the container-receiving position 211, the container output position 212, the first injection position 213, and the second injection position 214.

The container-positioning table 21 is not limited to rotary motion. In another preferred embodiment (not shown in figures), the container-positioning table 21 moves the container holder 22 along a straight line or a bent line to move the container holder 22 among multiple specific positions. Moreover, the container-positioning table 21 is not limited to move each container holder 22 to four different positions; in a less-automated embodiment of the cell passaging device, that is, the container-positioning table 21 only moves the container holder 22 to the first injection position 213.

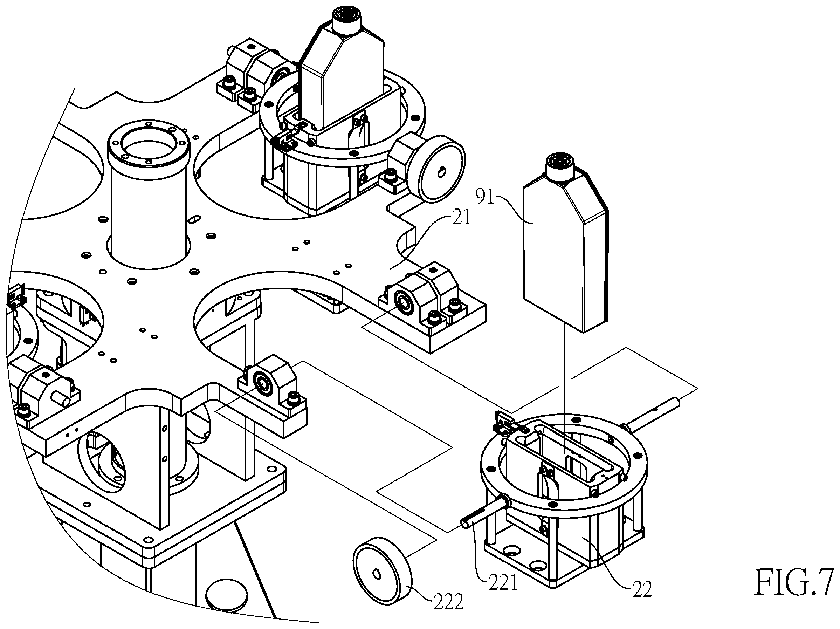

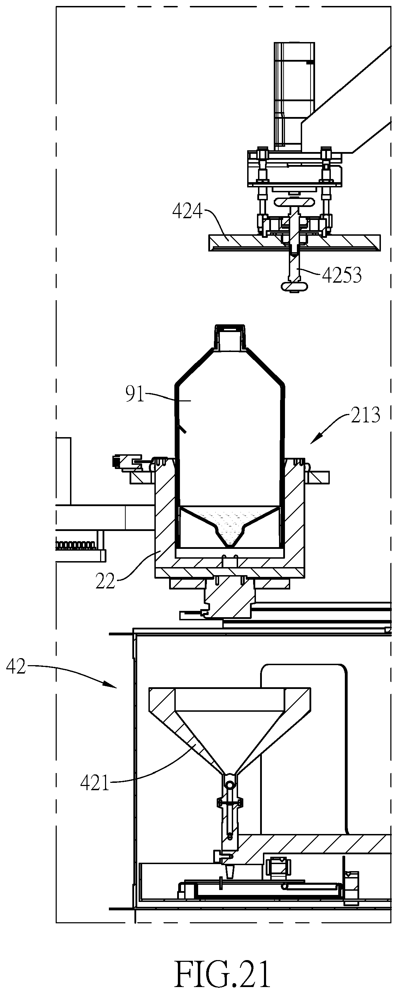

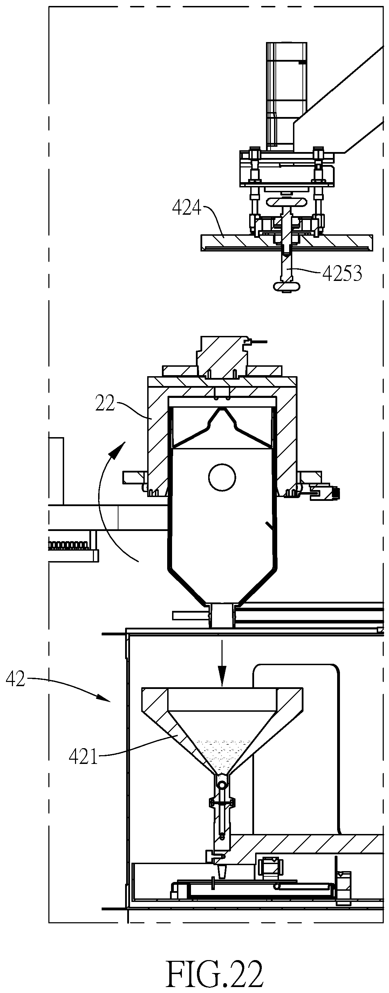

With reference to Figs. 7, 21 and 22, each container holder 22 is configured to accommodate one of the cell culture containers 91 or one of the centrifuge containers 92. Each of the container holders 22 is pivotally mounted on the container-positioning table 21, and is pivotable to an emptying angle (as shown in Fig. 22) to empty contents in the culture container 91 or the centrifuge container 92. To be precise, each container holder 22 is mounted on the container-positioning table 21 via a shaft 221 and is pivotable around the shaft 221.The shaft 221 is preferably horizontal.

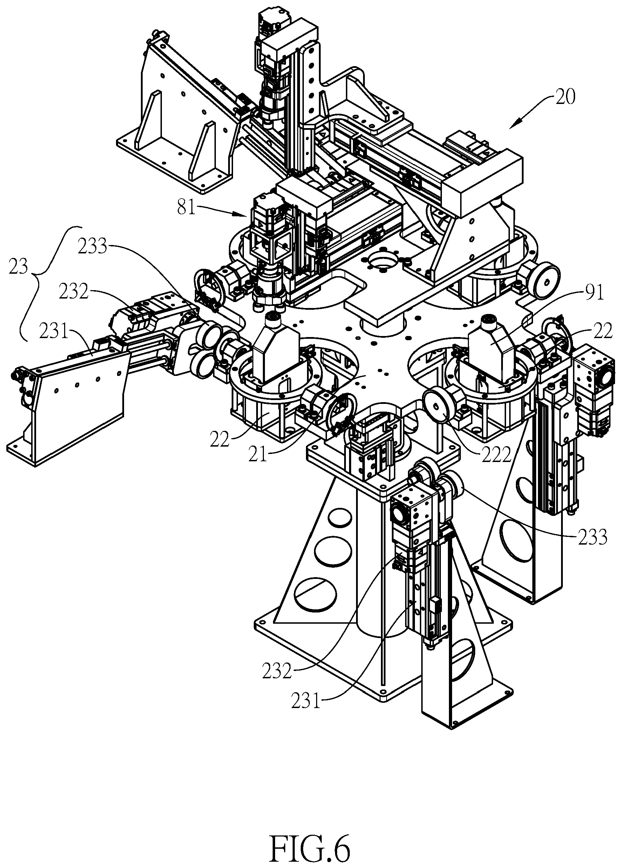

With reference to Figs. 6, 7, and 13, a number of the container-driving assemblies 23 equals the number of the container holders 22. Each of the container-driving assemblies 23 controls an angle of a respective one of the container holders 22 and is configured to rotate the respective one of the container holders 22 to the emptying angle or to sway the respective one of the container holders 22.

In the preferred embodiment, each container-driving assembly 23 is mounted on the base and has a linear module 231, a motor 232, and one or multiple driving wheels 233. The linear module 231 is mounted on the base 10. The motor 232 and the driving wheels 233 are mounted on a slider of the linear module 231.

A position of said slider is controllable and the slider is configured to move toward the container holder 22 to make the driving wheels 233 abut against a driven wheel 222. The driven wheel 222 is connected to the shaft 221 such that the motor 232 can control the angle of the corresponding container holder 22 via the driving wheels 233 and the driven wheel 222. By mounting the container-driving assembly 23 on the base 10 instead of the container-positioning table 21, makes cabling of the linear module 231 and motor 232 more simplified.

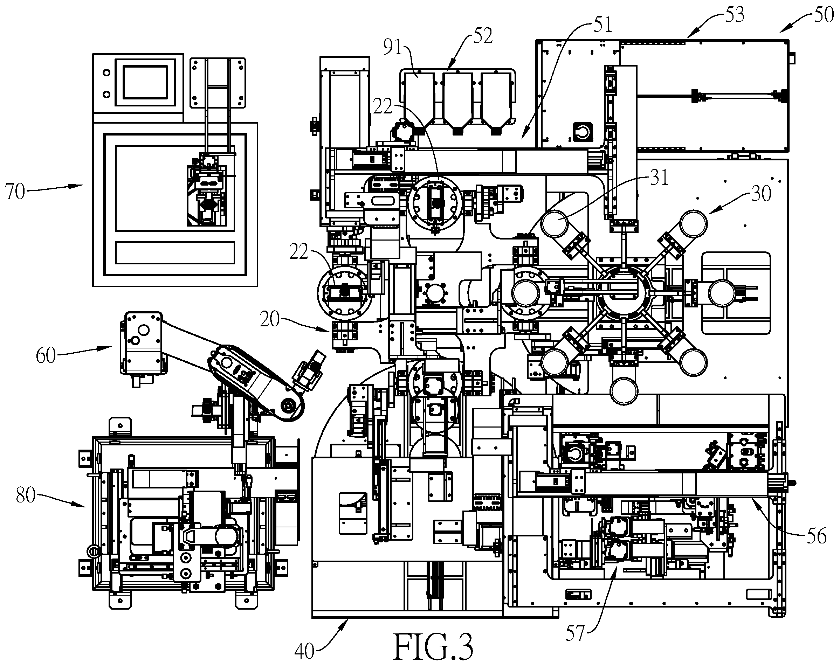

With reference to Figs 3, 4, and 12, the liquid solution injection device 30 is mounted on the base 10 and has multiple injection heads 31. Each of the injection heads 31 is connected to a container with a kind of liquid solution and is movable to a position above the second injection position 214 to inject the liquid solution in a connected container into the cell culture container 91 located in the container holder 22 at the second injection position 214. The liquid solution injection device 30 is preferably disposed on a side, which is toward the second injection position 214, of the container-handling device 20.

In the preferred embodiment, the liquid solution injection device 30 is a turret. The injection heads 31 are disposed around a center axis of the turret and the turret is configured to rotate a specified injection head 31 to the position above the second injection position 214 for liquid solution injection. The liquid solutions connected to the injection head 31 include kinds of liquid reagents which can make adherent cells detach from a surface of the cell culture container 91, and also include balance reagent which can neutralize the effectiveness of the detaching reagent.

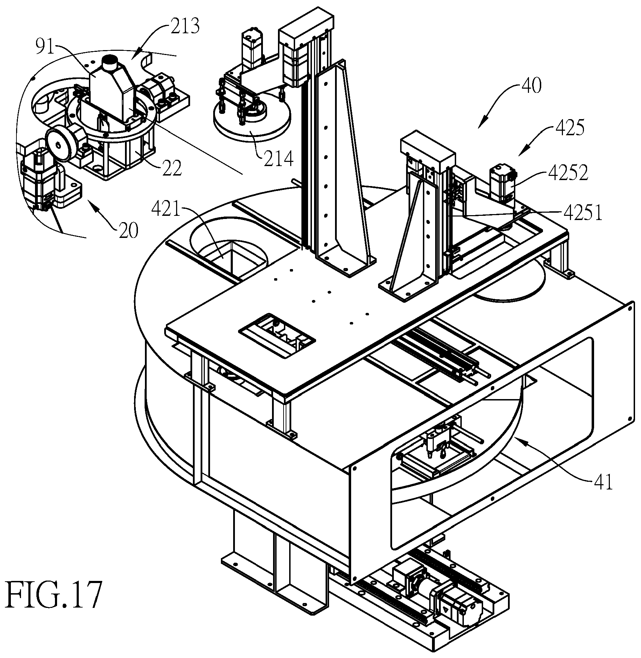

With reference to Figs. 3, 4, 17 and 18, the liquid equal-splitting device 40 is disposed on a side of the container-handling device 20; the liquid equal-splitting device 40 has a funnel-positioning table 41 and at least one gravitational liquid-splitting device 42. The funnel-positioning table 41 is rotatably mounted on the base 10, and is preferably a conventional rotary indexing table.

The funnel-positioning table 41 is disposed lower in height than the container-positioning table 21, but disposed higher in height than the position of two centrifuge containers 92. One indexed position of the funnel-positioning table 41 is located under the first injection position 213 of the container-positioning table 21, and another indexed position of the funnel-positioning table 41 is located above the position of two centrifuge containers 92.

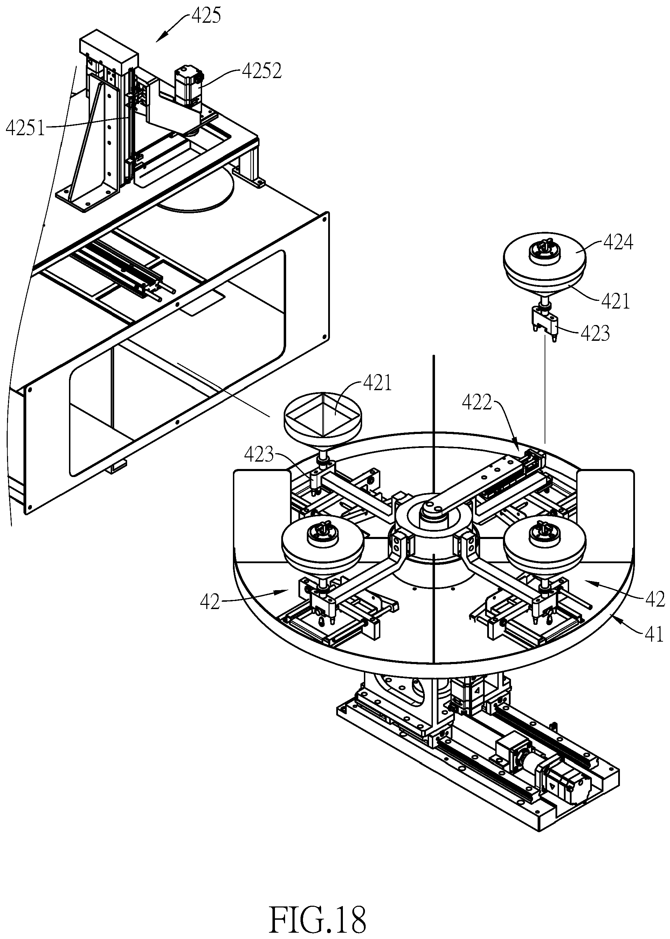

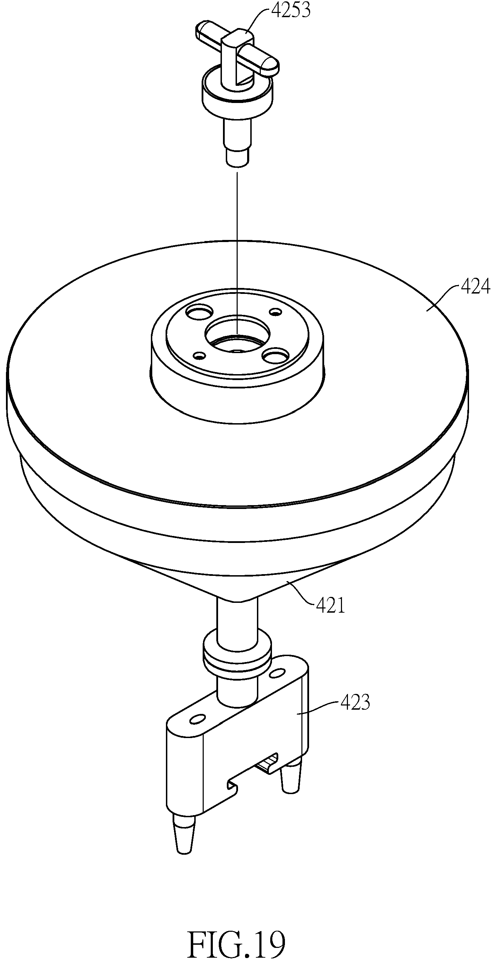

With reference to Figs. 18 to 20, the number of the gravitational liquid-splitting device 42 is preferably four, at least one, and the four gravitational liquid-splitting devices 42 are disposed around a center axis of the funnel-positioning table 41. Each gravitational liquid-splitting device 42 has a funnel 421, a dispensing valve 422, a liquid-splitting part 423, and a stirring assembly 425. In the preferred embodiment, each gravitational liquid-splitting devices 42 further has a funnel cover 424. The funnel 421 has an inner space and a bottom opening, and the inner space is connected to the bottom opening. A first ferromagnetic part 4221 of the dispensing valve 422 is mounted in the funnel 421 and is configured to close or open the bottom opening of the funnel 421. Here "closing the bottom opening" means the dispensing valve 422 prevents liquid in the funnel 421 from flowing down via the bottom opening, and therefore the funnel 421 dispensing valve 422 does not have to directly cover the bottom opening.

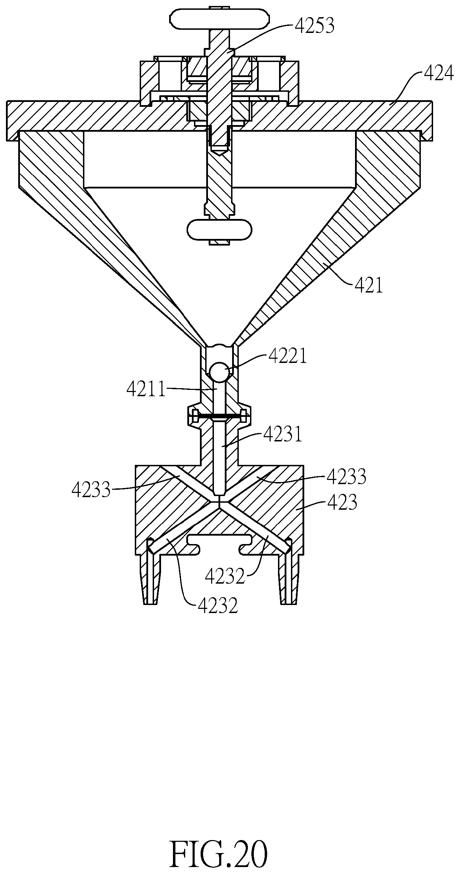

In the preferred embodiment, the inner space of the funnel 421 forms a channel 4211 (as shown in Fig. 20) in the bottom of the funnel 421. The channel 4211 extends upward and downward, and a lower end of the channel 4211 forms the bottom opening of the funnel 421. The first ferromagnetic part 4221 of the dispensing valve 422 is disposed in the channel 4211 and is configured to be controlled to close or open the channel 4211, thereby closing or opening the bottom opening of the funnel 421.

With reference to Figs. 20 to 23 and Fig. 25, the dispensing valve 422 has the first ferromagnetic part 4221, a valve actuator 4222, and a second ferromagnetic part 4223. The first ferromagnetic part 4221 is movably disposed in the channel 4211 of the funnel 421 and driven by a weight of the first ferromagnetic part 4221 to move toward the bottom opening of the funnel 421 to close the bottom opening (as shown in Fig. 20). The first ferromagnetic part 4221 is preferably a ball with a metal core.

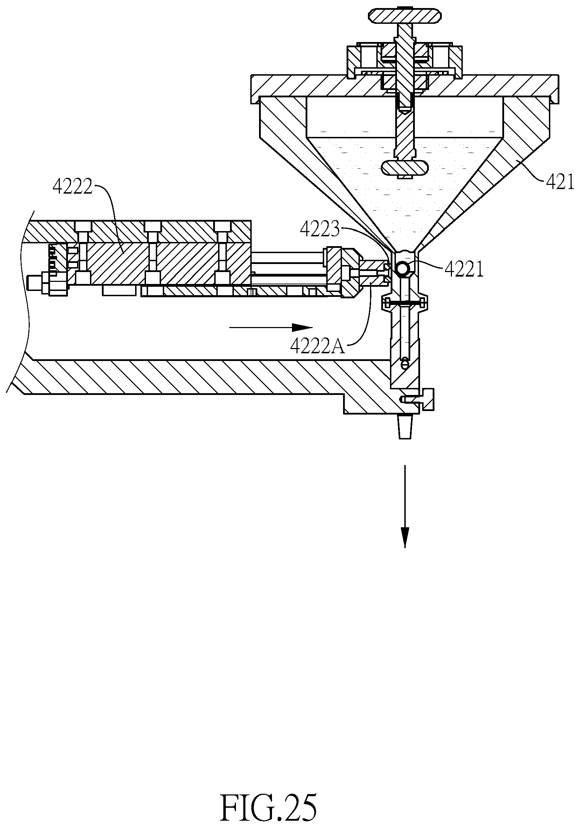

The valve actuator 4222 is disposed outside of the funnel 421 and has a moving end 4222A which is controllable and configured to move toward the first ferromagnetic part 4221 to an open position (as shown in Fig. 25). To be precise, the valve actuator 4222 is a pneumatic cylinder. The valve actuator 4222 is mounted on the base 10 and can be controlled by pressurized air to make a slider of the valve actuator 4222 move toward the first ferromagnetic part 4221.

The second ferromagnetic part 4223 is mounted on a moving end 4222A of the valve actuator 4222. The second ferromagnetic part 4223 and the first ferromagnetic part 4221 magnetically attract or repel each other. When the moving end 4222A of the valve actuator 4222 is at the open position, the first ferromagnetic part 4221 is driven by the second ferromagnetic part 4223 to open the bottom opening of the funnel.

In the preferred embodiment, the second ferromagnetic part 4223 is a permanent magnet. When the moving end 4222A of the valve actuator 4222 is at the open position, the first ferromagnetic part 4221 is magnetically attracted by the second ferromagnetic part 4223 and opens the channel 4211. In another preferred embodiment (not shown in figures), the first ferromagnetic part 4221 and the second ferromagnetic part 4223 are permanent magnets which magnetically attract or repel each other, or the second ferromagnetic part 4223 is a coil which generates magnetic field when powered-on.

With reference to Figs. 18 to 20, the liquid-splitting part 423 is connected to the bottom of the funnel 421. The liquid-splitting part 423 has an input channel 4231, two output channels 4232, and two auxiliary channels 4233 formed in the liquid-splitting part 423, but the auxiliary channels can be omitted.

The input channel 4231 extends upward and downward; an upper end of the input channel 4231 forms an input opening on a top of the liquid-splitting part 423 and connected to the bottom opening of the funnel. Each of the output channels 4232 has a first end and a second end; the first end is connected to a lower end of the input channel 4231; the second end extends inclinedly downward and connected to an exterior of the liquid-splitting part.

Liquid entering the input channel 4231 is split equally between the two output channels 4232 by gravity and shapes of the output channels 4232. In the preferred embodiment, each output channel 4232 is linear, and its lower end is connected to the exterior of the liquid-splitting part via a vertical channel.

Each auxiliary channel 4233 has a third end and a fourth end; the third end is connected to one of the two output channels 4232; the fourth end extends inclinedly upward and is connected to the exterior of the liquid-splitting part. To be specific, each auxiliary channel 4233 extends along a same straight line with a respective one of the output channels 4232.

Function of the auxiliary channels 4233 is to balance pressure in the two output channels 4232, thereby stabilizing flow speed in the two output channels 4232. Therefore, the auxiliary channels 4233 further ensure the liquid entering the input channel 4231 is split equally between the two output channels 4232. Moreover, each auxiliary channel 4233 is in line with a respective one of the output channels 4232 for ease of cleaning. In another preferred embodiment, there is only one auxiliary channel 4233, and the third end of the auxiliary channel 4233 is connected to both of the two output channels 4232.

With reference to Figs. 18, 20, 23, and 24, the funnel cover 424 detachably covers an upper opening of the funnel 421. The stirring assembly 425 has a linear module 4251, a stirring motor 4252, and a stirrer 4253. The linear module 4251 is mounted on the base 10 and can be controlled to move a slider 4251A of the linear module 4251. The stirring motor 4252 is mounted on the slider 4251A of the linear module 4251. The stirrer 4253 is rotatably mounted through the funnel cover 424 and is configured to stir liquid in the funnel 421.

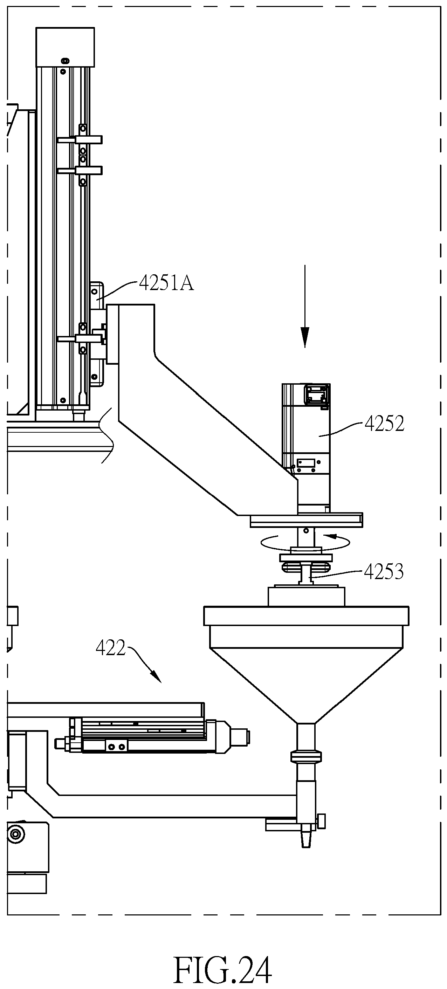

When the funnel cover 424 covers the funnel 421, the linear module 4251 is configured to move the stirring motor 4252 toward the stirrer 4253 (as shown in Fig. 24) such that an output axle of the stirring motor 4252 is connected to an upper end of the stirrer 4253, thereby allowing the stirring motor 4252 to rotate the stirrer 4253. Structure of the stirring assembly 425 is not limited by the abovementioned as long as there is a stirrer 4253 configured to stir liquid in the funnel 421.

With reference to Figs. 3, 4, 18, and 20, rotation of the funnel-positioning table 41 is configured to move each of the gravitational liquid-splitting devices 42 from a position under the first injection position 213 to a position above the position of two centrifuge containers 92 such that liquid in the funnel 421 of each gravitational liquid-splitting device 42 flows into the two centrifuge containers 92 via the two output channels 4232 respectively.

In the preferred embodiment, the funnel-positioning table 41 is both rotatable on a horizontal plane and linearly movable toward or away from the container-handling device 20. The funnel-positioning table 41 moves each gravitational liquid-splitting device 42 to the position above the two centrifuge containers 92 by a combination of said rotation and linear motion.

Operation mode of the funnel-positioning table 41 is not limited to rotation. In another preferred embodiment, the funnel-positioning table 41 moves the gravitational liquid-splitting devices 42 along a straight line or a bent line to move the gravitational liquid-splitting devices 42 among multiple specific positions.

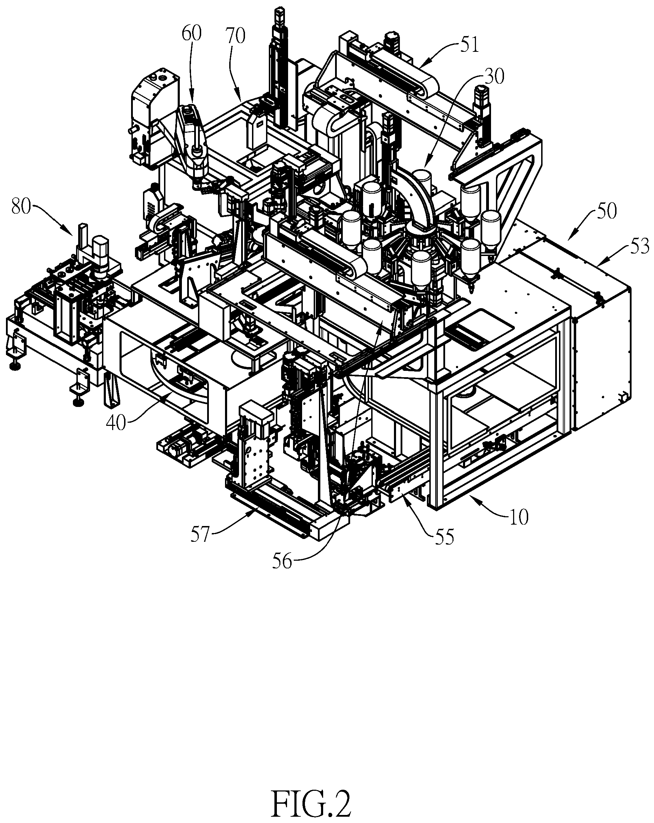

With reference to Figs. 2 to 4 and Fig. 28, the primary container-moving device 50 is configured to move each of the two centrifuge containers 92 from the position under the gravitational liquid-splitting device 42 to the container holder 22 at the container-receiving position 211.

Additionally, the primary container-moving device 50 is also configured to move the cell culture container 91 in the container holder 22 at the container-receiving position 211 to the position under the gravitational liquid-splitting devices 42 such that liquid in the funnel 421 of the corresponding gravitational liquid-splitting device 42 flows into the cell culture container 91. Therefore, the primary container-moving device 50 allows the present invention to recycle two of the cell culture containers 91 so that they can be used again as the two centrifuge containers 92.

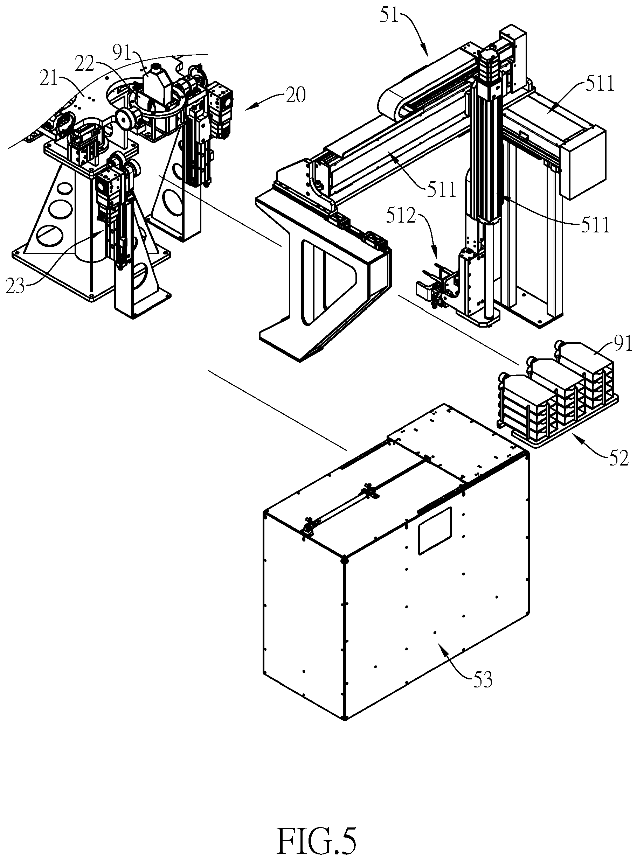

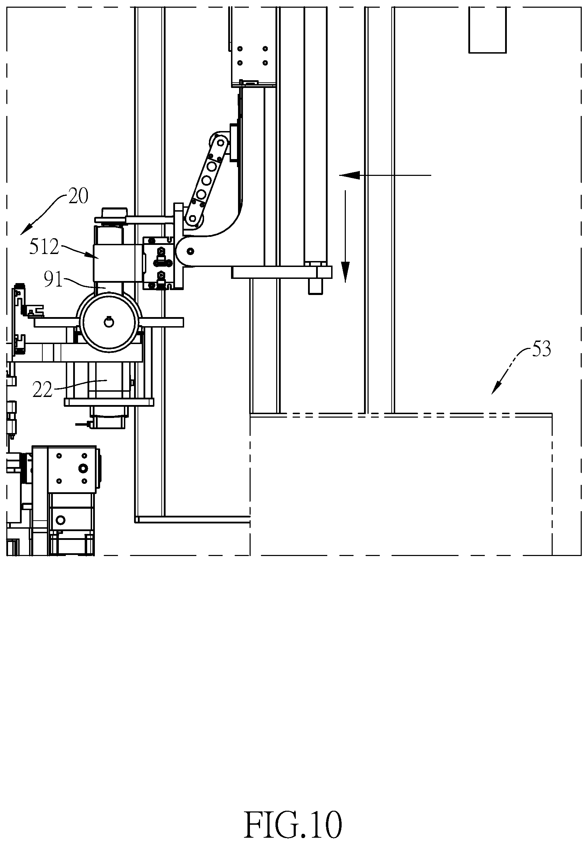

In the preferred embodiment, the primary container-moving device 50 has a first multi-axis transfer mechanism 51, a container rack 52, a first conveyer 53, a second conveyer 54, a third conveyer 55, a second multi-axis transfer mechanism 56, and a bottling and transferring mechanism 57.
With reference to Fig. 5 and Figs. 8 to 10, the first multi-axis transfer mechanism 51 is preferably a three-axis transfer mechanism which includes three linear modules 511. One of the linear modules 511 that extends vertically has a gripper 512 mounted on a lower end of said linear module 511. The gripper 512 is configured to grip one of the containers (e.g., the cell culture container 91 or the centrifuge container 92) accommodated in the container holder 22 at the container-receiving position 211, the container rack 52, or the first conveyer 53. The gripper 512 is also configured to put the gripped container in any one of the said three components. To be more precise, the gripper 512 is configured to rotate 90 degrees along a horizontal axis to change an angle of the gripped container.

The container rack 52 is configured to accommodate twelve containers to improve operational efficiency. The first conveyer 53 includes a translating mechanism and an elevating mechanism such that the first conveyer 53 moves containers from a bottom to a top of the first conveyer 53 where the first multi-axis transfer mechanism 51 can grip the container or put several new containers in the container rack 52 one by one.

The second conveyer 54 and the third conveyer 55 are linear belt conveyors. One end of each conveyor is mounted in a bottom of the first conveyer 53, and another end of each conveyor extends to the second multi-axis transfer mechanism 56.

The second multi-axis transfer mechanism 56 is preferably a three-axis transfer mechanism which is configured to move containers among the second conveyer 54, the third conveyer 55, and the bottling and transferring mechanism 57.

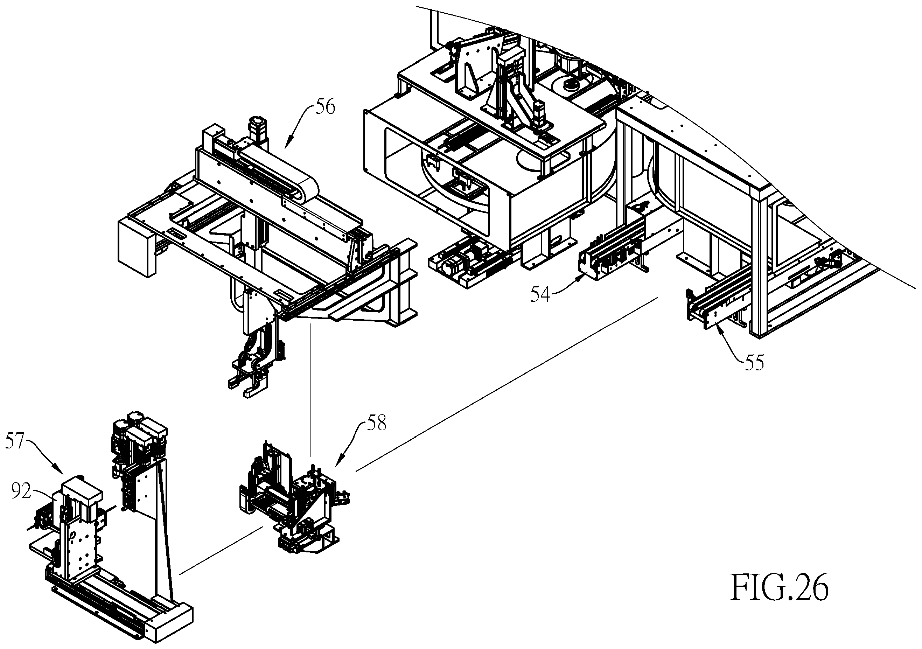

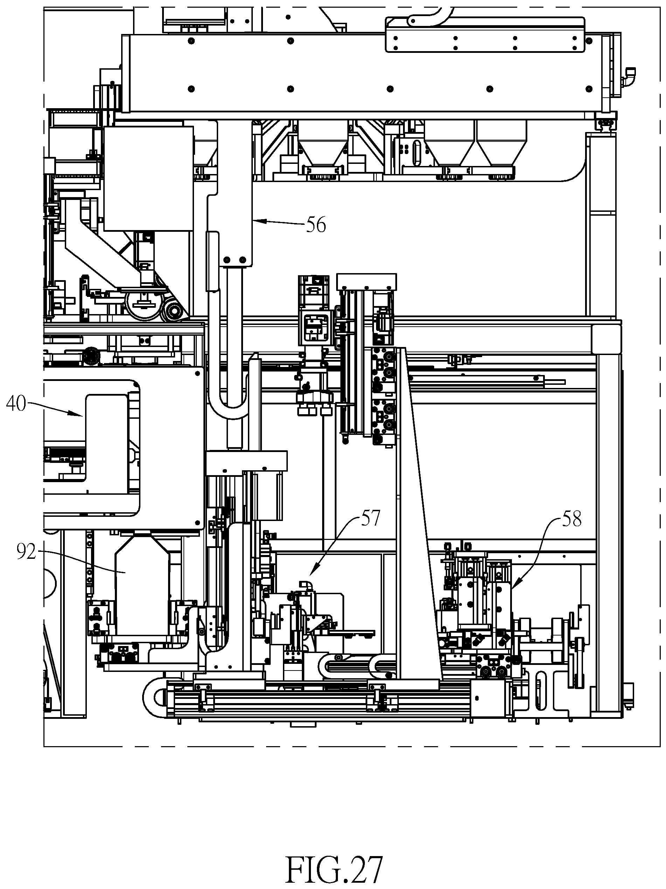

With reference to Figs. 26 to 28, the bottling and transferring mechanism 57 is configured to grip the double centrifuge containers 92, double the cell culture containers 91, double cryovials (not shown in figures), double cell solution containers (not shown in figures), or other types of containers. The bottling and transferring mechanism 57 is configured to align the two gripped containers with the two output channels 4232 of one of the gravitational liquid-splitting devices 42 such that the liquid in said gravitational liquid-splitting device 42 can be transferred to the two gripped containers. After transferring the liquid, the bottling and transferring mechanism 57 moves the two gripped containers away from the position under the container-positioning table 21 for the second multi-axis transfer mechanism 56 to grip for the further operation.

In the preferred embodiment, a cell solution container cap mechanism 58 is disposed under the second multi-axis transfer mechanism 56. The cell solution container cap mechanism 58 is configured to handle caps of the cell solution containers in collaboration with the second multi-axis transfer mechanism 56.

With reference to Figs. 2 to 4, the auxiliary container-moving device 60, the centrifuge 70, and the optical inspection device 80 are disposed on a same side of the container-handling device 20 as where the container output position 212 is. The centrifuge 70 and the optical inspection device 80 are standardized conventional equipment. The optical inspection device 80 is configured to measure a number of cells in the culture container 91 in order to determine whether a total number of the cells collected in the culture container 91 is satisfactory for the further cell passaging process. To be more specific, the optical inspection device 80 is configured to calculate automatically the number of detached cells in the cell culture containers 91 by a specific AI algorithm.

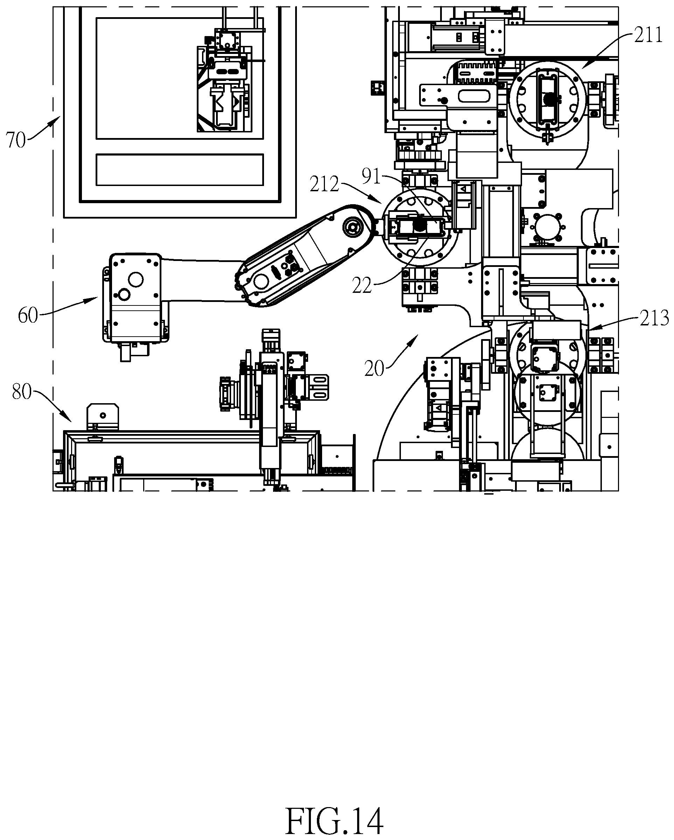

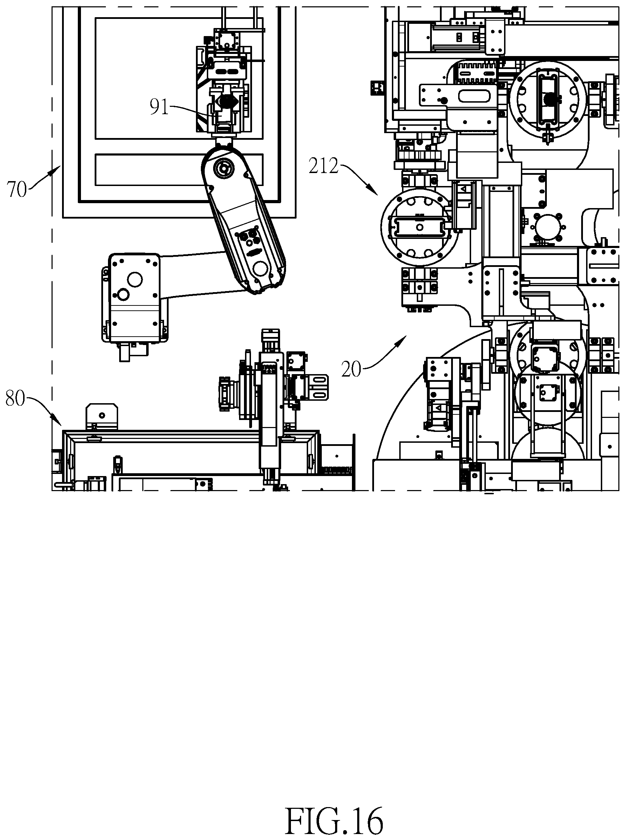

With reference to Figs. 14 to 16, the auxiliary container-moving device 60 is configured to move the centrifuge container 92 or the cell culture container 91 in the container holder 22 at the container output position 212 into the centrifuge 70, and also configured to move the centrifuge container 92 or the cell culture container 91 in the centrifuge 70 back into the container holder 22 at the container output position 212.

Additionally, the auxiliary container-moving device 60 is configured to move the centrifuge container 92 or the cell culture container 91 in the container holder 22 at the container output position 212 into the optical inspection device 80, and also configured to move the centrifuge container 92 or the cell culture container 91 in the optical inspection device 80 back into the container holder 22 at the container output position 212. The auxiliary container-moving device 60 is preferably a robotic arm.

The cell passaging method in accordance with the present invention is preferably performed using the abovementioned cell passaging device. The cell passaging device is configured to automatically perform various cell passaging methods for cell subculture process. One of the cell passaging methods for subculture of adherent cells, which is in accordance with the present invention, is explained below.

With reference to Figs. 3 to 5, multiple cell culture containers 91 already in the stationary phase are transmitted to the first conveyer 53 of the primary container-moving device 50 by an external transfer mechanism.

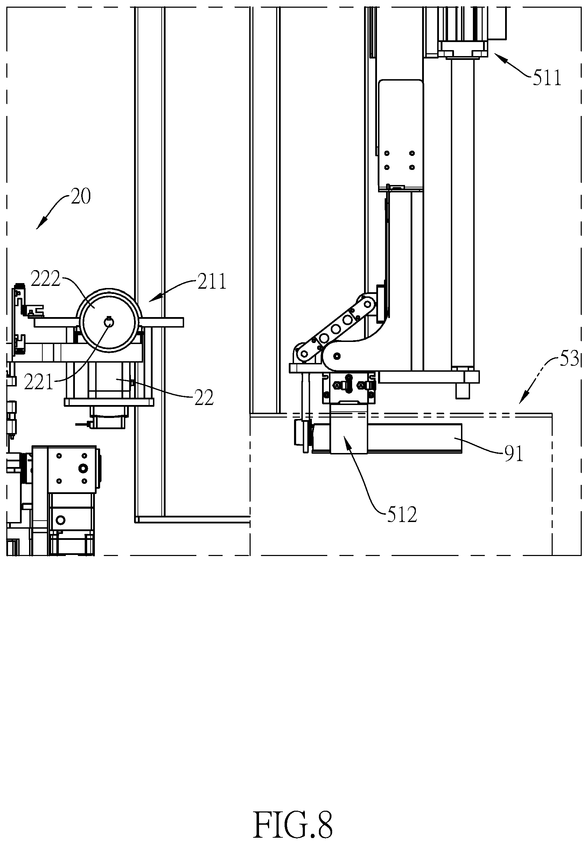

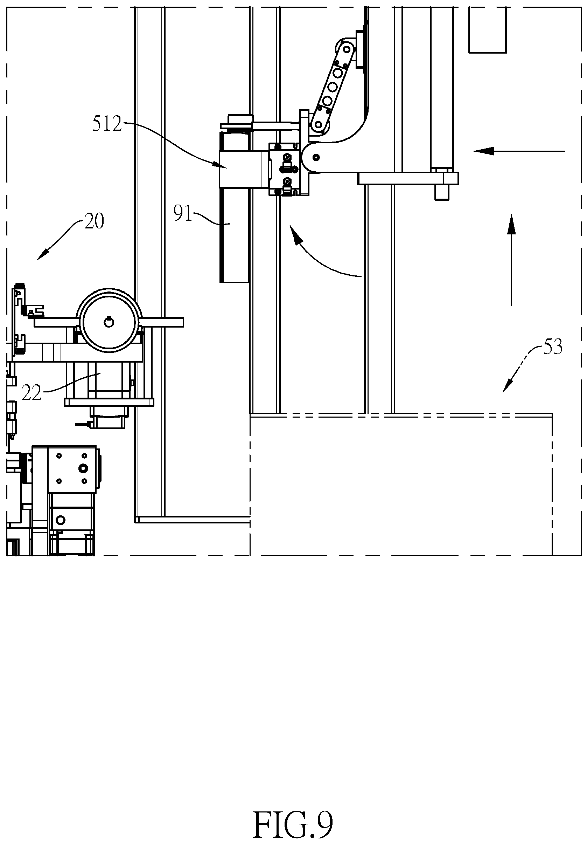

With reference to Figs. 8 to 10, then, the first multi-axis transfer mechanism 51 grips the cell culture containers 91 in the first conveyer 53 and dispenses the cell culture containers 91 into the container holder 22 at the container-receiving position 211. The gripper 512 of the first multi-axis transfer mechanism 51 rotates the gripped cell culture container 91 from a horizontal posture to a vertical posture (as shown in Figs. 8 and 9) before putting the gripped cell culture container 91 into the container holder 22 for ease of subsequent operation.

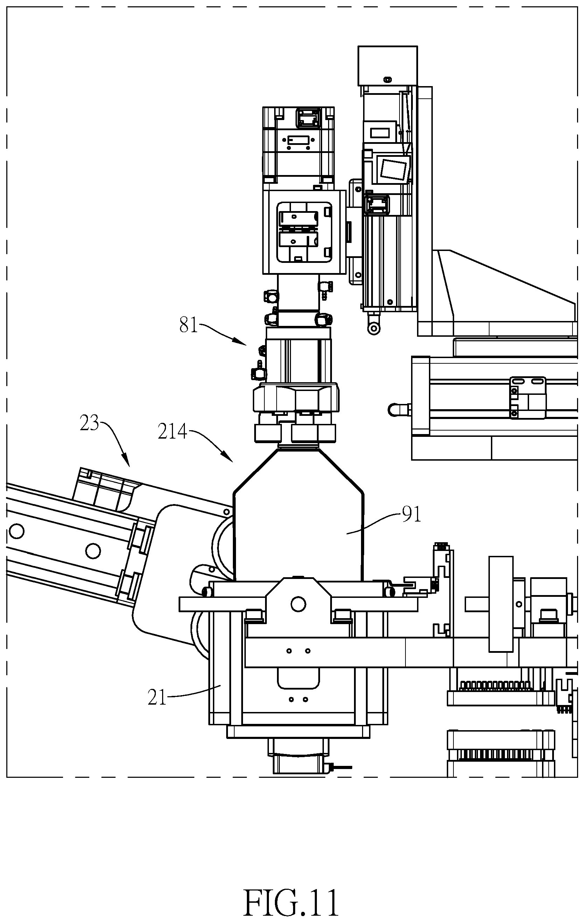

With reference to Figs. 4 and 11, then, the cell culture container 91 is moved from the container-receiving position 211 to the second injection position 214. A cap mechanism 81 moves down to grip a cap of the cell culture container 91.

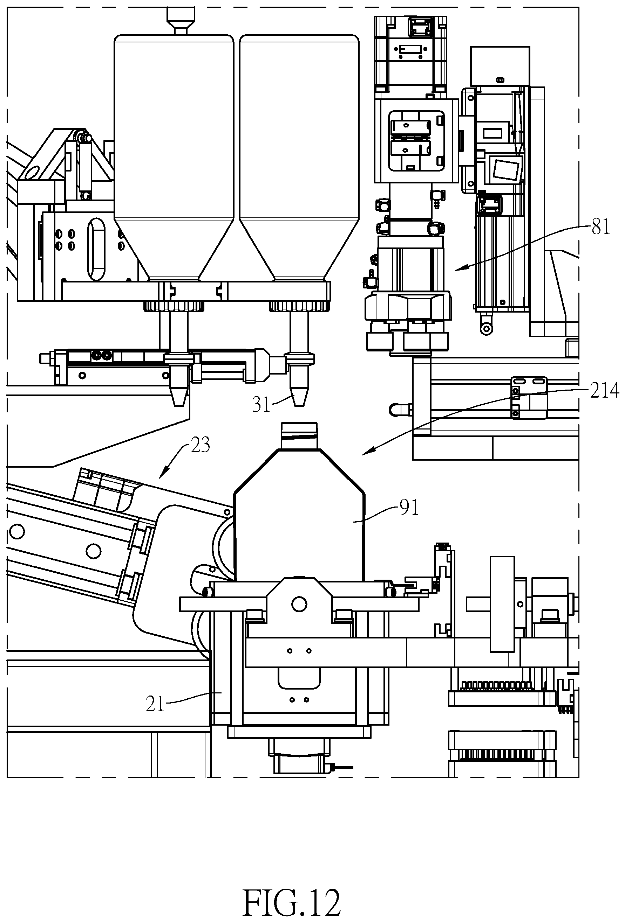

With reference to Fig. 12, then, a cap mechanism 81 loosens the cap of the cell culture container 91 and moves the cap aside, and the container-driving assembly 23 rotates the container holder 22 to empty the content (ex. medium) of the cell culture container 91. Then, the injection head 31 of the liquid solution injection device 30 moves to the position above the second injection position 214 and injects the detaching reagent (ex. Trypsin) into the cell culture container 91, wherein the detaching reagent is functioned to make the adherent cells detach from the surface of the cell culture container 91.

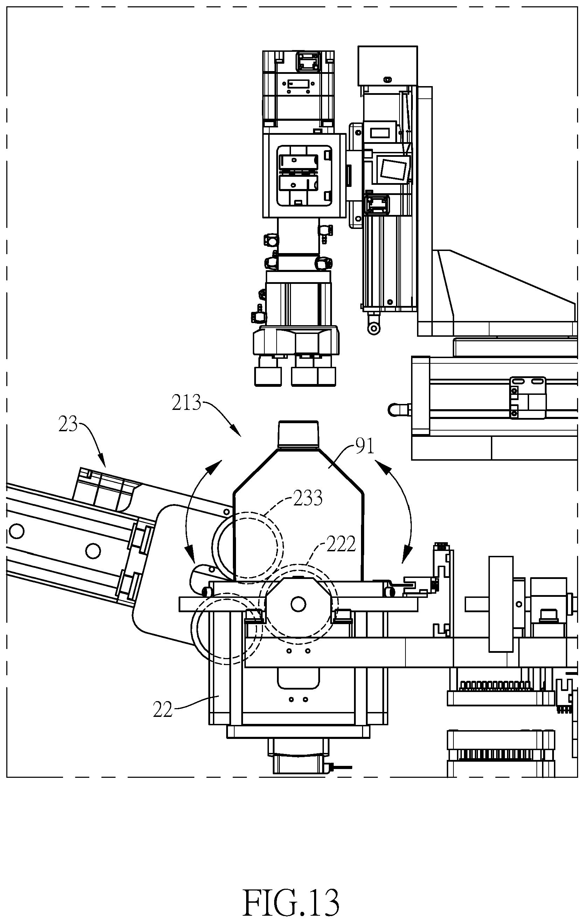

With reference to Figs. 4 and 13, then, the cap mechanism 81 puts the cap back onto the cell culture container 91 and tightens the cap, and the culture container 91 is moved to the first injection position 213. The container-driving assembly 23 sways the culture container 91 at the first injection position 213 to make cells detached from the surface of the cell culture container 91.

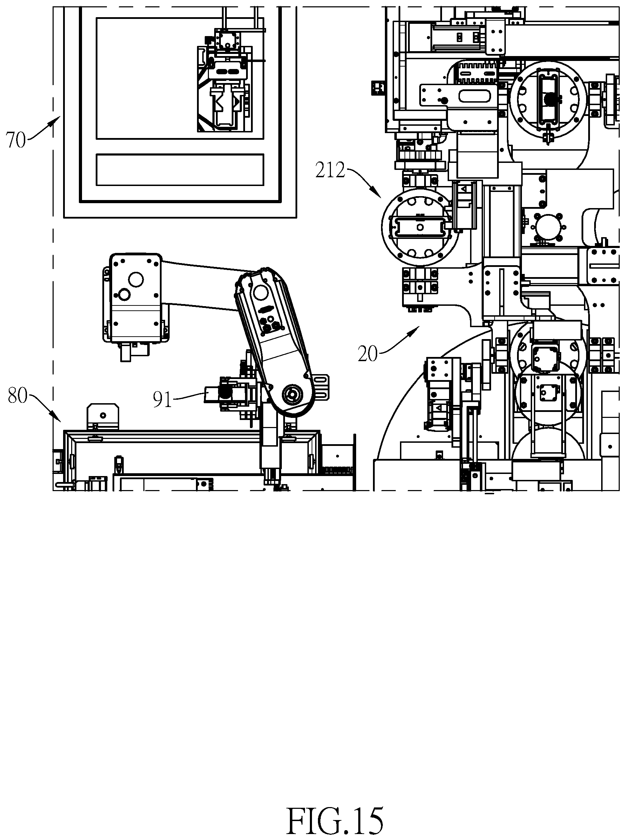

With reference to Figs. 14 and 15, then, the cell culture container 91 is moved to the container output position 212, and then the auxiliary container-moving device 60 grips the cell culture container 91 and dispenses the culture container 91 into the optical inspection device 80 to calculate automatically the number of cells in the cell culture container 91 by using a specific AI algorithm.

When the process of cell counting in the optical inspection device 80 has finished, the culture cell container 91 is moved back to the container output position 212, and subsequently moved to the second injection position 214. At the second injection position 214, another one of the injection heads 31 of the liquid solution injection device 30 injects balance reagent into the cell culture container 91 to neutralize the effectiveness of detaching reagent.

With reference to Figs. 4, 21, and 22, then, the culture cell container 91 is moved to the first injection position 213, and the container-driving assembly 23 rotates the container holder 22 to pour contents (e.g., solution with detached cells) in the cell culture container 91 into the funnel 421 of the gravitational liquid-splitting device 42. The funnel cover 424 on top of the funnel 421 has been removed before this step. Then, the above steps are performed again for the rest of the cell culture containers 91 until the solutions with detached cells from all culture containers 91 are collected in the funnel 421.

Among the cell culture containers 91, the first two cell culture containers 91 are moved to the position under one of the gravitational liquid-splitting devices 42 after their solutions with detached cells are poured into the funnel 421 such that the first two cell culture containers 91 serve as the two centrifuge containers 92.

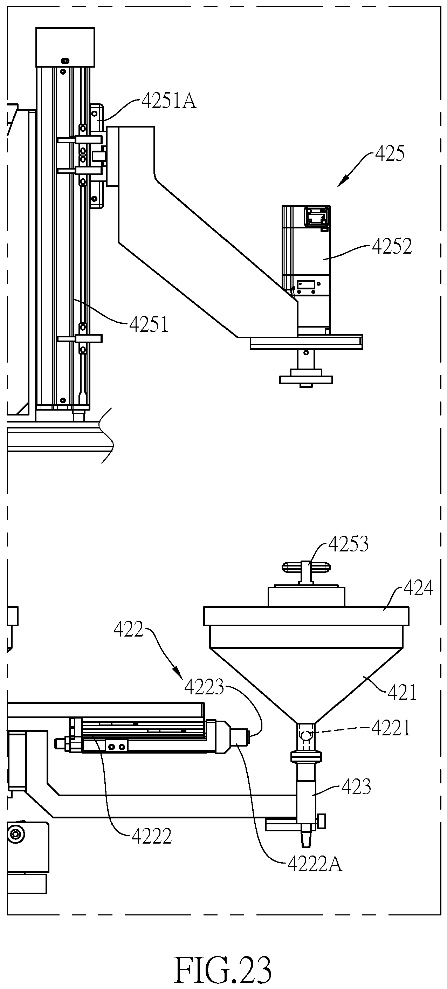

With reference to Figs. 23 and 24, after the solutions with detached cells from all cell culture containers 91 are collected in the funnel 421, the funnel-positioning table 41 moves the gravitational liquid-splitting device 42 clockwise to the position above the two centrifuge containers 92, and meanwhile the slider 4251A of the stirring assembly 425 moves down to connect the stirring motor 4252 to the upper end of the stirrer 4253. The stirring motor 4252 drives the stirrer 4253 to keep stirring the solution with detached cells in the funnel 421.

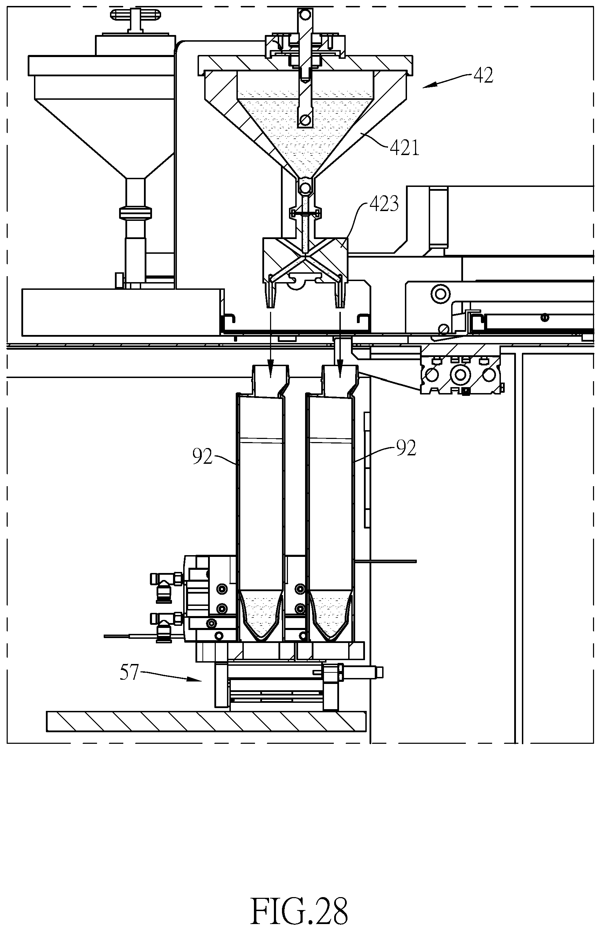

With reference to Figs. 25 and 28, then, the valve actuator 4222 of the dispensing valve 422 moves the second ferromagnetic part 4223 toward the first ferromagnetic part 4221 to the open position to move the first ferromagnetic part 4221 such that the solution with detached cells in the funnel 421 is split equally between the two centrifuge containers 92.

With reference to Fig. 4, finally, the two centrifuge containers 92 are moved to the container-handling device 20 by the primary container-moving device 50, and then moved into the centrifuge 70 by the auxiliary container-moving device 60 to perform centrifugation. After the centrifugation, the two centrifuge containers 92 are ready for subsequent cell passaging operations.

The cell passaging method in accordance with the present invention is not limited to aforementioned as long as contents in the cell culture containers 91 are split equally between the two centrifuge containers 92 by using the gravitational liquid-splitting device 42, and then the two centrifuge containers 92 are disposed oppositely in the centrifuge 70 to perform centrifugation to separate the cells from the solution.

In addition to performing the abovementioned cell passaging method, the cell passaging device is also configured to automatically perform various processes related to cell culture processing, such as primary specimen cells extraction process, cell culture container medium exchange process, cell culture container optical inspection process, and some of the cell cryopreservation processes such as cryovial dispensing, partial cell thawing process, and cell solution bottling for shipping process.

In summary, liquid (solution with detached cells) in the funnel 421 automatically flows downward into the centrifuge containers 92 due to gravity because the liquid-splitting part 423 of the gravitational liquid-splitting device 42 is disposed under the funnel 421, the input channel 4231 extends upward and downward, and meanwhile the output channels 4232 extend inclinedly downward; therefore, no liquid residue is left in the funnel 421, the input channel 4231, and the output channel 4232.

As a result, the present invention prevents loss of cells during transferring operation, avoids damage to the cells under pressurization by using pump, and effectively automates the process of collecting solution with detached cells together and then split the collected solution with detached cells equally between the two centrifuge containers 92, thereby improving efficiency of cell passaging and reducing cost of cell passaging.

Moreover, with the stirrer 4253 of the gravitational liquid-splitting device 42 continuously stirring the solution with detached cells in the funnel, 421, the present invention prevents cells from clustering together or adhering to surfaces of the funnel 421 or channels, thereby keeping flow rates in the two output channels 4232 remain equal and ensuring the solution with detached cells is split equally between the two centrifuge containers 92.

Finally, the cell passaging device replaces traditional manual shaking operation by automatically swaying the cell culture container 91, transferring the solution with detached cells into the gravitational liquid-splitting device 42, and splitting the solution with detached cells equally between the two centrifuge containers 92, thereby reducing labor-intensity for the cell passaging operation. As a result, the present invention has advantages such as high efficiency, stable quality, and reducing risk of contamination in cell passaging processes.

## Claims

1. A gravitational liquid-splitting device (42) **characterized in that** the gravitational liquid-splitting device (42) comprises:
a funnel (421) having an inner space and a bottom opening connected to the inner space;
a dispensing valve (422) mounted to the funnel (421) and configured to close or open the bottom opening of the funnel (421);
a liquid-splitting part (423) connected to a bottom of the funnel (421) and having:
an input channel (4231) formed in the liquid-splitting part (423) and extending upward and downward; an upper end of the input channel (4231) forming an input opening on a top of the liquid-splitting part (423) and connected to the bottom opening of the funnel (421); and
two output channels (4232) formed in the liquid-splitting part (423); each of the output channels (4232) having a first end and a second end; the first end connected to a lower end of the input channel (4231); the second end extending inclinedly downward and connected to an exterior of the liquid-splitting part (423); and
a stirring assembly (425) having
a funnel (421) cover detachably covering an upper opening of the funnel (421); and
a stirrer (4253) connected to the funnel (421) cover and configured to stir liquid in the funnel (421).

2. The gravitational liquid-splitting device (42) as claimed in claim 1, wherein the liquid-splitting part (423) has at least one auxiliary channel (4233) formed in the liquid-splitting part (423) and having:
a third end connected to one of the two output channels (4232); and
a fourth end extending inclinedly upward and connected to the exterior of the liquid-splitting part (423) .

3. The gravitational liquid-splitting device (42) as claimed in claim 2, wherein the at least one auxiliary channel (4233) includes two auxiliary channels (4233); each of the two auxiliary channels (4233) is connected to a respective one of the two output channels (4232) and extends along a same straight line with said output channel (4232).

4. The gravitational liquid-splitting device (42) as claimed in any one of claims 1 to 3, wherein the dispensing valve (422) has:
a first ferromagnetic part (4221) movably disposed in the inner space of the funnel (421) and driven by a weight of the first ferromagnetic part (4221) to move toward the bottom opening of the funnel (421) to close the bottom opening;
a valve actuator (4222) disposed outside of the funnel (421); a moving end (4222A) of the valve actuator (4222) being movable toward the first ferromagnetic part (4221) to an open position;
a second ferromagnetic part (4223) mounted to the moving end (4222A) of the valve actuator (4222); the second ferromagnetic part (4223) and the first ferromagnetic part (4221) magnetically attracting or repelling each other;
wherein, when the moving end (4222A) of the valve actuator (4222) is at the open position, the first ferromagnetic part (4221) is driven by the second ferromagnetic part (4223) to open the bottom opening of the funnel (421).

5. A cell passaging device, configured to concentrate contents collected from multiple cell culture containers (91) into two centrifuge containers (92); the cell passaging device **characterized in that** the cell passaging device comprises:
a base (10);
a container-handling device (20) mounted on the base (10) and having:
a container-positioning table (21) rotatably or movably mounted on the base (10);
multiple container holders (22) pivotally mounted on the container-positioning table (21); each of the container holders (22) configured to accommodate one of the cell culture containers (91) or one of the centrifuge containers (92), and being pivotable to an emptying angle to empty contents in the cell culture container (91) or the centrifuge container (92); and
multiple container-driving assemblies (23); each of the container-driving assemblies (23) controlling an angle of a respective one of the container holders (22) and configured to rotate the respective one of the container holders (22) to the emptying angle or to sway the respective one of the container holders (22);
wherein, the container-positioning table (21) is configured to move each of the container holders (22) to a first injection position (213); and
a liquid equal-splitting device (40) disposed on a side of the container-handling device (20); the liquid equal-splitting device (40) having:
a funnel-positioning table (41) rotatably or movably mounted on the base (10); and
at least one said gravitational liquid-splitting device (42) as claimed in any one of claims 1 to 4 mounted on the funnel-positioning table (41);
wherein, the funnel-positioning table (41) is configured to move the at least one gravitational liquid-splitting device (42) from a position under the first injection position (213) to a position above the two centrifuge containers (92) such that the liquid in the funnel (421) of the at least one gravitational liquid-splitting device (42) flows into the two centrifuge containers (92) via the two output channels (4232) respectively.

6. The cell passaging device as claimed in claim 5, wherein:
the container-positioning table (21) is configured to move each of the container holders (22) to a second injection position (214); and
the cell passaging device has a liquid solution injection device (30); the liquid solution injection device (30) is mounted on the base (10) and has multiple injection heads (31); each of the injection heads (31) is connected to a container with a kind of liquid solution and is movable to a position above the second injection position (214) to inject the liquid solution in the container into the cell culture container (91) located in the container holder (22) in the second injection position (214).

7. The cell passaging device as claimed in claim 5, wherein:
the container-positioning table (21) of the container-handling device (20) is configured to move each of the container holders (22) to a container-receiving position (211) or a container output position (212);
the cell passaging device has:
a primary container-moving device (50) configured to move each of the two centrifuge containers (92) from the position under the at least one gravitational liquid-splitting device (42) to the container holder (22) at the container-receiving position (211);
a centrifuge disposed on a side of the container-handling device (20); and
an auxiliary container-moving device configured to move the centrifuge container (92) in the container holder (22) at the container output position (212) into the centrifuge, and configured to move the centrifuge container (92) in the centrifuge back into the container holder (22) at the container output position (212).

8. The cell passaging device as claimed in claim 5, wherein:
the container-positioning table (21) of the container-handling device (20) is configured to move each of the container holders (22) to a container-receiving position (211) or a container output position (212);
the cell passaging device has:
an optical inspection device (80) disposed on a side of the container-handling device (20) and configured to calculate the number of detached cells in the cell culture containers (91); and
an auxiliary container-moving device (60) configured to move the cell culture container (91) in the container holder (22) at the container output position (212) into the optical inspection device (80), and configured to move the cell culture container (91) in the optical inspection device (80) back into the container holder (22) at the container output position (212).

9. The cell passaging device as claimed in claim 7, wherein:
the container-positioning table (21) of the container-handling device (20) is rotatably mounted on the base (10) and configured to move each of the container holders (22) to circulate between the container-receiving position (211), the container output position (212), the first injection position (213), and a second injection position (214);
the cell passaging device has:
a liquid solution injection device (30) mounted on the base (10) and having multiple injection heads (31); each of the injection heads (31) connected to a container with a kind of liquid solution and being movable to a position above the second injection position (214) to inject the liquid solution in the container into the cell culture container (91) located in the container holder (22) at the second injection position (214); and
an optical inspection device (80) disposed on a side of the container-handling device (20) and configured to calculate the number of detached cells in the cell culture containers (91); and
the auxiliary container-moving device (60) is configured to move the cell culture container (91) in the container holder (22) at the container output position (212) into the optical inspection device (80), and configured to move the cell culture container (91) in the optical inspection device (80) back into the container holder (22) at the container output position (212);
wherein, the second injection position (214) and the container output position (212) are oppositely disposed on the container-handling device (20); the centrifuge, the auxiliary container-moving device (60), and the optical inspection device (80) are disposed on a side, which is toward the container output position (212), of the container-handling device (20); the liquid solution injection device (30) is disposed on a side, which is toward the second injection position (214), of the container-handling device (20).

10. The cell passaging device as claimed in claim 5, wherein:
the container-positioning table (21) of the container-handling device (20) is configured to move each of the container holders (22) to a container-receiving position (211) or a container output position (212); and
the cell passaging device has a primary container-moving device (50); the primary container-moving device (50) is configured to move the cell culture container (91) in the container holder (22) at the container-receiving position (211) to the position under the at least one gravitational liquid-splitting device (42) such that the liquid in the funnel (421) of the at least one gravitational liquid-splitting device (42) flows into the cell culture container (91).

11. A cell passaging method for concentrating contents collected from multiple cell culture containers (91) into two centrifuge containers (92); the cell passaging method **characterized in that** the cell passaging method comprises steps of: using the gravitational liquid-splitting device (42) as claimed in any one of claims 1 to 4 to split contents in the cell culture containers (91) equally between the two centrifuge containers (92), then disposing the two centrifuge containers (92) oppositely in a centrifuge and using the centrifuge to perform centrifugation.

## Patentansprüche

1. Gravitationsgesteuerte Flüssigkeitsteilvorrichtung (42), **dadurch gekennzeichnet, dass** die gravitationsgesteuerte Flüssigkeitsteilvorrichtung (42) Folgendes aufweist:
einen Trichter (421) mit einem Innenraum und einer Bodenöffnung, die mit dem Innenraum verbunden ist;
ein Auslassventil (422), das an dem Trichter (421) angebracht ist und so konfiguriert ist, die Bodenöffnung des Trichters (421) zu öffnen oder zu schließen;
ein Flüssigkeitsteilmodul (423), das mit einem Boden des Trichters (421) verbunden ist und Folgendes aufweist:
einen Eingabekanal (4231), der im Flüssigkeitsteilmodul (423) ausgebildet ist und sich nach oben und unten erstreckt; wobei ein oberes Ende des Eingabekanals (4231) eine Eingabeöffnung an der Oberseite des Flüssigkeitsteilmoduls (423) bildet und mit der Bodenöffnung des Trichters (421) verbunden ist; und
zwei Ausgabekanäle (4232), die im Flüssigkeitsteilmodul (423) ausgebildet sind; wobei jeder der Ausgabekanäle (4232) ein erstes Ende und ein zweites Ende hat; wobei das erste Ende mit einem unteren Ende des Eingabekanals (4231) verbunden ist; wobei sich das zweite Ende schräg nach unten erstreckt und mit einem Außenbereich des Flüssigkeitsteilmoduls (423) verbunden ist; und
eine Rühreinheit (425), die Folgendes aufweist:
eine Abdeckung für den Trichter (421), die eine obere Öffnung des Trichters (421) abnehmbar abdeckt; und
einen Rührer (4253), der mit der Abdeckung für den Trichter (421) verbunden ist und so konfiguriert ist, Flüssigkeit im Trichter (421) zu rühren.

2. Gravitationsgesteuerte Flüssigkeitsteilvorrichtung (42) nach Anspruch 1, wobei das Flüssigkeitsteilmodul (423) mindestens einen Hilfskanal (4233) aufweist, der im Flüssigkeitsteilmodul (423) ausgebildet ist und Folgendes aufweist:
ein drittes Ende, das mit einem der beiden Ausgabekanäle (4232) verbunden ist; und
ein viertes Ende, das sich schräg nach oben erstreckt und mit einem Außenbereich des Flüssigkeitsteilmoduls (423) verbunden ist.

3. Gravitationsgesteuerte Flüssigkeitsteilvorrichtung (42) nach Anspruch 2, wobei der mindestens eine Hilfskanal (4233) zwei Hilfskanäle (4233) aufweist; wobei jeder der zwei Hilfskanäle (4233) mit einem der beiden Ausgabekanäle (4232) verbunden ist und entlang einer geraden Linie mit dem jeweiligen Ausgabekanal (4232) verläuft.

4. Gravitationsgesteuerte Flüssigkeitsteilvorrichtung (42) nach einem der Ansprüche 1 bis 3, wobei das Auslassventil (422) Folgendes aufweist:
ein erstes ferromagnetisches Element (4221), das beweglich im Innenraum des Trichters (421) angeordnet ist und durch das Gewicht des ersten ferromagnetischen Elements (4221) dazu angetrieben wird, sich in Richtung der Bodenöffnung des Trichters (421) zu bewegen, um die Bodenöffnung zu schließen;
einen Ventilantrieb (4222), der außerhalb des Trichters (421) angebracht ist; ein bewegliches Ende (4222A) des Ventilantriebs (4222) ist beweglich in Richtung des ersten ferromagnetischen Elements (4221) zu einer offenen Position;
ein zweites ferromagnetisches Element (4223), das am beweglichen Ende (4222A) des Ventilantriebs (4222) angebracht ist; wobei sich das zweite ferromagnetische Element (4223) und das erste ferromagnetische Element (4221) magnetisch anziehen oder abstoßen;
wobei, wenn sich das bewegliche Ende (4222A) des Ventilantriebs (4222) in der offenen Position befindet, das erste ferromagnetische Element (4221) durch das zweite ferromagnetische Element (4223) dazu angetrieben wird, die Bodenöffnung des Trichters (421) zu öffnen.

5. Zellpassiervorrichtung, die konfiguriert ist, um Inhalte aus mehreren Zellkulturbehältern (91) in zwei Zentrifugenbehälter (92) zu konzentrieren; wobei die Zellpassiervorrichtung **dadurch gekennzeichnet ist, dass** sie Folgendes aufweist:
eine Basis (10);
eine Behälterhandhabungseinheit (20), die auf der Basis (10) angebracht ist und Folgendes aufweist:
einen Behälterpositioniertisch (21), der drehbar oder beweglich auf der Basis (10) montiert ist;
mehrere Behälterhalterungen (22), die schwenkbar am Behälterpositioniertisch (21) montiert sind; wobei jede der Behälterhalterungen (22) so konfiguriert ist, einen der Zellkulturbehälter (91) oder einen der Zentrifugenbehälter (92) aufzunehmen und in einem Entleerungswinkel geschwenkt werden kann, um Inhalte im Zellkulturbehälter (91) oder im Zentrifugenbehälter (92) zu entleeren; und
mehrere Behälterantriebseinheiten (23); jede der Behälterantriebseinheiten (23) steuert einen Winkel einer entsprechenden Behälterhalterung (22) und ist konfiguriert, die entsprechende Behälterhalterung (22) in den Entleerungswinkel zu drehen oder die entsprechende Behälterhalterung (22) zu schwenken;
eine Flüssigkeitsgleichverteilungsvorrichtung (40), die an einer Seite der Behälterhandhabungseinheit (20) angebracht ist; wobei die Flüssigkeitsgleichverteilungsvorrichtung (40) Folgendes aufweist:
einen Trichterpositioniertisch (41), der drehbar oder beweglich auf der Basis (10) montiert ist; und
mindestens eine der in einem der Ansprüche 1 bis 4 genannten gravitationsgesteuerten Flüssigkeitsteilvorrichtungen (42), die an dem Trichterpositionstisch (41) montiert ist; wobei der Trichterpositioniertisch (41) so konfiguriert ist, die mindestens eine gravitationsgesteuerte Flüssigkeitsteilvorrichtung (42) von einer Position unter der ersten Injektionsposition (213) zu einer Position über den beiden Zentrifugenbehältern (92) zu bewegen, so dass die Flüssigkeit im Trichter (421) der mindestens einen gravitationsgesteuerten Flüssigkeitsteilvorrichtung (42) über die beiden Ausgabekanäle (4232) in die beiden Zentrifugenbehälter (92) fließt.

6. Zellpassiervorrichtung nach Anspruch 5, wobei:
der Behälterpositioniertisch (21) so konfiguriert ist, jede der Behälterhalterungen (22) zu einer zweiten Injektionsposition (214) zu bewegen; und
die Zellpassiervorrichtung eine Flüssigkeitseinheit (30) aufweist; wobei die Flüssigkeitseinheit (30) auf der Basis (10) montiert ist und mehrere Injektionsköpfe (31) hat; wobei jeder der Injektionsköpfe (31) mit einem Behälter verbunden ist, der eine Art von Flüssigkeit enthält, und beweglich zu einer Position über der zweiten Injektionsposition (214) ist, um die Flüssigkeit aus dem Behälter in den Zellkulturbehälter (91) zu injizieren, der sich in der Behälterhalterung (22) an der zweiten Injektionsposition (214) befindet.

7. Zellpassiervorrichtung nach Anspruch 5, wobei:
der Behälterpositioniertisch (21) der Behälterhandhabungseinheit (20) so konfiguriert ist, jede der Behälterhalterungen (22) zu einer Behälteraufnahmeposition (211) oder einer Behälterausgabeposition (212) zu bewegen;
wobei die Zellpassiervorrichtung Folgendes aufweist:
eine Primärbehälterbewegungseinheit (50), die so konfiguriert ist, jeden der beiden Zentrifugenbehälter (92) von der Position unter der mindestens einen gravitationsgesteuerten Flüssigkeitsteilvorrichtung (42) zur Behälterhalterung (22) an der Behälteraufnahmeposition (211) zu bewegen;
eine Zentrifuge, die an einer Seite der Behälterhandhabungseinheit (20) angebracht ist; und
eine Hilfsbehälterbewegungseinheit (60), die so konfiguriert ist, den Zentrifugenbehälter (92) in der Behälterhalterung (22) an der Behälterausgabeposition (212) in die Zentrifuge zu bewegen und den Zentrifugenbehälter (92) aus der Zentrifuge zurück in die Behälterhalterung (22) an der Behälterausgabeposition (212) zu bewegen.

8. Zellpassiervorrichtung nach Anspruch **5,** wobei:
der Behälterpositioniertisch (21) der Behälterhandhabungseinheit (20) so konfiguriert ist, jede der Behälterhalterungen (22) zu einer Behälteraufnahmeposition (211) oder einer Behälterausgabeposition (212) zu bewegen; und
wobei die Zellpassiervorrichtung Folgendes aufweist:
eine optische Inspektionseinheit (80), die an einer Seite der Behälterhandhabungseinheit (20) angebracht ist und so konfiguriert ist, die Anzahl der abgelösten Zellen in den Zellkulturbehältern (91) zu berechnen; und
eine Hilfsbehälterbewegungseinheit (60), die so konfiguriert ist, den Zellkulturbehälter (91) in der Behälterhalterung (22) an der Behälterausgabeposition (212) in die optische Inspektionseinheit (80) zu bewegen und den Zellkulturbehälter (91) aus der optischen Inspektionseinheit (80) zurück in die Behälterhalterung (22) an der Behälterausgabeposition (212) zu bewegen.

9. Zellpassiervorrichtung nach Anspruch 7, wobei:
der Behälterpositioniertisch (21) der Behälterhandhabungseinheit (20) drehbar auf der Basis (10) montiert ist und so konfiguriert ist, jede der Behälterhalterungen (22) zwischen der Behälteraufnahmeposition (211), der Behälterausgabeposition (212), der ersten Injektionsposition (213) und einer zweiten Injektionsposition (214) zu bewegen;
wobei die Zellpassiervorrichtung Folgendes aufweist:
eine Flüssigkeitseinheit (30), die auf der Basis (10) montiert ist und mehrere Injektionsköpfe (31) aufweist; wobei jeder der Injektionsköpfe (31) mit einem Behälter verbunden ist, der eine Art von Flüssigkeit enthält, und zu einer Position über der zweiten Injektionsposition (214) beweglich ist, um die Flüssigkeit aus dem Behälter in den Zellkulturbehälter (91) zu injizieren, der sich in der Behälterhalterung (22) an der zweiten Injektionsposition (214) befindet; und
eine optische Inspektionseinheit (80), die an einer Seite der Behälterhandhabungseinheit (20) angebracht ist und so konfiguriert ist, die Anzahl der abgelösten Zellen in den Zellkulturbehältern (91) zu berechnen.

10. Zellpassiervorrichtung nach Anspruch 5, wobei:
der Behälterpositioniertisch (21) der Behälterhandhabungseinheit (20) so konfiguriert ist, jede der Behälterhalterungen (22) zu einer Behälteraufnahmeposition (211) oder einer Behälterausgabeposition (212) zu bewegen; und
die Zellpassiervorrichtung umfasst: eine Primärbehälterbewegungseinheit (50), die so konfiguriert ist, den Zellkulturbehälter (91) in der Behälterhalterung (22) an der Behälteraufnahmeposition (211) zu der Position unter der mindestens einen gravitationsgesteuerten Flüssigkeitsteilvorrichtung (42) zu bewegen, so dass die Flüssigkeit im Trichter (421) der mindestens einen gravitationsgesteuerten Flüssigkeitsteilvorrichtung (42) in den Zellkulturbehälter (91) fließt.

11. Zellpassierverfahren zum Konzentrieren von Inhalten, die aus mehreren Zellkulturbehältern (91) gesammelt wurden, in zwei Zentrifugenbehälter (92); wobei das Zellpassierverfahren **dadurch gekennzeichnet ist, dass** es folgende Schritte aufweist:
Verwenden der gravitationsgesteuerten Flüssigkeitsteilvorrichtung (42) nach einem der Ansprüche 1 bis 4, um Inhalte in den Zellkulturbehältern (91) gleichmäßig auf die beiden Zentrifugenbehälter (92) zu verteilen; anschließendes Anordnen der beiden Zentrifugenbehälter (92) gegenüberliegend in einer Zentrifuge und Durchführen einer Zentrifugation.

## Revendications

1. Dispositif de séparation de liquide par gravité (42) **caractérisé en ce que** le dispositif de séparation de liquide par gravité (42) comprend :
un entonnoir (421) ayant un espace interne et une ouverture de fond raccordée à l'espace interne ;
une vanne de distribution (422) montée sur l'entonnoir (421) et configurée pour fermer ou ouvrir l'ouverture de fond de l'entonnoir (421) ;
une partie de séparation de liquide (423) raccordée à un fond de l'entonnoir (421) et ayant :
un canal d'entrée (4231) formé dans la partie de séparation de liquide (423) et s'étendant vers le haut et vers le bas ; une extrémité supérieure du canal d'entrée (4231) formant une ouverture d'entrée sur un dessus de la partie de séparation de liquide (423) et étant raccordée à l'ouverture de fond de l'entonnoir (421) ; et
deux canaux de sortie (4232) formés dans la partie de séparation de liquide (423) ; chacun des canaux de sortie (4232) ayant une première extrémité et une deuxième extrémité ; la première extrémité étant raccordée à une extrémité inférieure du canal d'entrée (4231) ; la deuxième extrémité s'étendant de manière inclinée vers le bas et étant raccordée à un extérieur de la partie de séparation de liquide (423) ; et
un ensemble d'agitation (425) ayant
un couvercle d'entonnoir (421) recouvrant de manière détachable une ouverture supérieure de l'entonnoir (421) ; et
un agitateur (4253) raccordé au couvercle d'entonnoir (421) et configuré pour agiter un liquide dans l'entonnoir (421).

2. Dispositif de séparation de liquide par gravité (42) selon la revendication 1, dans lequel la partie de séparation de liquide (423) possède au moins un canal auxiliaire (4233) formé dans la partie de séparation de liquide (423) et ayant :
une troisième extrémité raccordée à l'un des deux canaux de sortie (4232) ; et
une quatrième extrémité s'étendant de manière inclinée vers le haut et raccordée à l'extérieur de la partie de séparation de liquide (423).

3. Dispositif de séparation de liquide par gravité (42) selon la revendication 2, dans lequel l'au moins un canal auxiliaire (4233) comporte deux canaux auxiliaires (4233) ; chacun des deux canaux auxiliaires (4233) est raccordé à un canal respectif des deux canaux de sortie (4232) et s'étend le long d'une même ligne droite avec ledit canal de sortie (4232).

4. Dispositif de séparation de liquide par gravité (42) selon l'une quelconque des revendications 1 à 3, dans lequel la vanne de distribution (422) possède :
une première partie ferromagnétique (4221) disposée de manière mobile dans l'espace interne de l'entonnoir (421) et entraînée par un poids de la première partie ferromagnétique (4221) afin qu'elle se déplace vers l'ouverture de fond de l'entonnoir (421) pour fermer l'ouverture de fond ;
un actionneur de vanne (4222) disposé à l'extérieur de l'entonnoir (421) ; une extrémité mobile (4222A) de l'actionneur de vanne (4222) étant mobile vers la première partie ferromagnétique (4221) jusqu'à une position ouverte ;
une seconde partie ferromagnétique (4223) montée sur l'extrémité mobile (4222A) de l'actionneur de vanne (4222) ; la seconde partie ferromagnétique (4223) et la première partie ferromagnétique (4221) s'attirant ou se repoussant mutuellement de manière magnétique ;
dans lequel, lorsque l'extrémité mobile (4222A) de l'actionneur de vanne (4222) est dans la position ouverte, la première partie ferromagnétique (4221) est entraînée par la seconde partie ferromagnétique (4223) pour ouvrir l'ouverture de fond de l'entonnoir (421).

5. Dispositif de passage de cellules, configuré pour concentrer des contenus collectés à partir de multiples récipients de culture cellulaire (91) dans deux récipients de centrifugeuse (92) ; le dispositif de passage de cellules étant **caractérisé en ce que** le dispositif de passage de cellules comprend :
une base (10) ;
un dispositif de manipulation de récipient (20) monté sur la base (10) et ayant :
une table de positionnement de récipient (21) montée de manière rotative ou mobile sur la base (10) ;
de multiples supports de récipient (22) montés de manière pivotante sur la table de positionnement de récipient (21) ; chacun des supports de récipient (22) étant configuré pour accueillir l'un des récipients de culture cellulaire (91) ou l'un des récipients de centrifugeuse (92), et étant pivotant jusqu'à un angle de vidage pour vider des contenus dans le récipient de culture cellulaire (91) ou le récipient de centrifugeuse (92) ; et
de multiples ensembles d'entraînement de récipient (23) ; chacun des ensembles d'entraînement de récipient (23) commandant un angle d'un support de récipient respectif parmi les supports de récipient (22) et étant configuré pour mettre en rotation le support de récipient respectif parmi les supports de récipient (22) jusqu'à l'angle de vidage ou pour faire basculer le support de récipient respectif parmi les supports de récipient (22) ;
dans lequel la table de positionnement de récipient (21) est configurée pour déplacer chacun des supports de récipient (22) jusqu'à une première position d'injection (213) ; et
un dispositif de séparation égale de liquide (40) disposé sur un côté du dispositif de manipulation de récipient (20) ; le dispositif de séparation égale de liquide (40) ayant :
une table de positionnement d'entonnoir (41) montée de manière rotative ou mobile sur la base (10) ; et
au moins un dit dispositif de séparation de liquide par gravité (42) selon l'une quelconque des revendications 1 à 4 monté sur la table de positionnement d'entonnoir (41) ;
dans lequel la table de positionnement d'entonnoir (41) est configurée pour déplacer l'au moins un dispositif de séparation de liquide par gravité (42) d'une position sous la première position d'injection (213) à une position au-dessus des deux récipients de centrifugeuse (92) de sorte que le liquide dans l'entonnoir (421) de l'au moins un dispositif de séparation de liquide par gravité (42) s'écoule dans les deux récipients de centrifugeuse (92) via les deux canaux de sortie (4232) respectivement.

6. Dispositif de passage de cellules selon la revendication 5, dans lequel :
la table de positionnement de récipient (21) est configurée pour déplacer chacun des supports de récipient (22) jusqu'à une seconde position d'injection (214) ; et
le dispositif de passage de cellules possède un dispositif d'injection de solution liquide (30) ; le dispositif d'injection de solution liquide (30) est monté sur la base (10) et possède de multiples têtes d'injection (31) ; chacune des têtes d'injection (31) est raccordée à un récipient avec un type de solution liquide et est mobile jusqu'à une position au-dessus de la seconde position d'injection (214) pour injecter la solution liquide du récipient dans le récipient de culture cellulaire (91) situé dans le support de récipient (22) dans la seconde position d'injection (214).

7. Dispositif de passage de cellules selon la revendication 5, dans lequel :
la table de positionnement de récipient (21) du dispositif de manipulation de récipient (20) est configurée pour déplacer chacun des supports de récipient (22) jusqu'à une position de réception de récipient (211) ou une position de sortie de récipient (212) ;
le dispositif de passage de cellules possède :
un dispositif primaire de déplacement de récipient (50) configuré pour déplacer chacun des deux récipients de centrifugeuse (92) de la position sous l'au moins un dispositif de séparation de liquide par gravité (42) jusqu'au support de récipient (22) à la position de réception de récipient (211) ;
une centrifugeuse disposée sur un côté du dispositif de manipulation de récipient (20) ; et
un dispositif auxiliaire de déplacement de récipient configuré pour déplacer le récipient de centrifugeuse (92) dans le support de récipient (22) à la position de sortie de récipient (212) jusque dans la centrifugeuse, et configuré pour remettre le récipient de centrifugeuse (92) de la centrifugeuse dans le support de récipient (22) à la position de sortie de récipient (212).

8. Dispositif de passage de cellules selon la revendication 5, dans lequel :
la table de positionnement de récipient (21) du dispositif de manipulation de récipient (20) est configurée pour déplacer chacun des supports de récipient (22) jusqu'à une position de réception de récipient (211) ou une position de sortie de récipient (212) ;
le dispositif de passage de cellules possède :
un dispositif d'inspection optique (80) disposé sur un côté du dispositif de manipulation de récipient (20) et configuré pour calculer le nombre de cellules détachées dans les récipients de culture cellulaire (91) ; et
un dispositif auxiliaire de déplacement de récipient (60) configuré pour déplacer le récipient de culture cellulaire (91) dans le support de récipient (22) à la position de sortie de récipient (212) jusque dans le dispositif d'inspection optique (80), et configuré pour remettre le récipient de culture cellulaire (91) du dispositif d'inspection optique (80) dans le support de récipient (22) à la position de sortie de récipient (212).

9. Dispositif de passage de cellules selon la revendication 7, dans lequel :
la table de positionnement de récipient (21) du dispositif de manipulation de récipient (20) est montée de manière rotative sur la base (10) et configurée pour déplacer chacun des supports de récipient (22) afin qu'ils circulent entre la position de réception de récipient (211), la position de sortie de récipient (212), la première position d'injection (213) et une seconde position d'injection (214) ;
le dispositif de passage de cellules possède :
un dispositif d'injection de solution liquide (30) monté sur la base (10) et ayant de multiples têtes d'injection (31) ; chacune des têtes d'injection (31) étant raccordée à un récipient avec un type de solution liquide et étant mobile jusqu'à une position au-dessus de la seconde position d'injection (214) pour injecter la solution liquide du récipient dans le récipient de culture cellulaire (91) situé dans le support de récipient (22) à la seconde position d'injection (214) ; et
un dispositif d'inspection optique (80) disposé sur un côté du dispositif de manipulation de récipient (20) et configuré pour calculer le nombre de cellules détachées dans les récipients de culture cellulaire (91) ; et
le dispositif auxiliaire de déplacement de récipient (60) est configuré pour déplacer le récipient de culture cellulaire (91) dans le support de récipient (22) à la position de sortie de récipient (212) jusque dans le dispositif d'inspection optique (80), et configuré pour remettre le récipient de culture cellulaire (91) du dispositif d'inspection optique (80) dans le support de récipient (22) à la position de sortie de récipient (212) ;
dans lequel la seconde position d'injection (214) et la position de sortie de récipient (212) sont disposées de manière opposée sur le dispositif de manipulation de récipient (20) ; la centrifugeuse, le dispositif auxiliaire de déplacement de récipient (60) et le dispositif d'inspection optique (80) sont disposés sur un côté, qui est vers la position de sortie de récipient (212), du dispositif de manipulation de récipient (20) ; le dispositif d'injection de solution liquide (30) est disposé sur un côté, qui est vers la seconde position d'injection (214), du dispositif de manipulation de récipient (20).

10. Dispositif de passage de cellules selon la revendication 5, dans lequel :
la table de positionnement de récipient (21) du dispositif de manipulation de récipient (20) est configurée pour déplacer chacun des supports de récipient (22) jusqu'à une position de réception de récipient (211) ou une position de sortie de récipient (212) ; et
le dispositif de passage cellulaire possède un dispositif primaire de déplacement de récipient (50) ; le dispositif primaire de déplacement de récipient (50) est configuré pour déplacer le récipient de culture cellulaire (91) dans le support de récipient (22) à la position de réception de récipient (211) jusqu'à la position sous l'au moins un dispositif de séparation de liquide par gravité (42) de sorte que le liquide dans l'entonnoir (421) de l'au moins un dispositif de séparation de liquide par gravité (42) s'écoule jusque dans le récipient de culture cellulaire (91).

11. Méthode de passage de cellules pour la concentration de contenus collectés à partir de multiples récipients de culture cellulaire (91) dans deux récipients de centrifugeuse (92) ; la méthode de passage de cellules étant **caractérisée en ce que** la méthode de passage de cellules comprend les étapes de : l'utilisation du dispositif de séparation de liquide par gravité (42) selon l'une quelconque des revendications 1 à 4 pour séparer des contenus dans les récipients de culture cellulaire (91) de manière égale entre les deux récipients de centrifugeuse (92), puis la disposition des deux récipients de centrifugeuse (92) de manière opposée dans une centrifugeuse et l'utilisation de la centrifugeuse pour réaliser une centrifugation.
